# EUROPEAN PATENT SPECIFICATION

(11) **EP 0 642 356 B1**
(45) Date of publication and mention of the grant of the patent: **16.04.2003**
(21) Application number: 93911098.7
(22) Date of filing: 04.05.1993
(51) Int. Cl.: A61K 39/395, C07K 16/28

(54) **ANTIBODIES TO P-SELECTIN AND THEIR USES**
ANTIKÖRPER GEGEN P-SELECTIN UND IHRE VERWENDUNG
ANTICORPS CONTRE LA P-SELECTINE ET LEURS EMPLOIS

(30) Priority: 05.05.1992 US 880196
(43) Date of publication of application: 15.03.1995
(73) Proprietor: Aeres Biomedical Limited, London NW7 1AD (GB)
(72) Inventor: CHESTNUT, Robert, W., Cardiff, CA 92007 (US); POLLEY, Margaret, J., La Jolla, CA 92037 (US); PAULSON, James, C., Del Mar, CA 92014 (US); BAYER, Robert, San Diego CA 92122 (US)
(74) Representative: Nicholls, Kathryn Margaret
(86) International application number: US9304274
(87) International publication number: WO93021956

(56) References cited:
- EP-A- 0 255 694
- WO-A-91/06632
- WO-A-92/01718
- US-A- 4 783 330
- GENG J.-G. ET AL.: "Rapid Neutrophil Adhesion to Activated Endothelium Mediated by GMP-140" NATURE, vol. 343, 22 February 1990, pages 757-760, XP000652356
- Nature, Volume 343, issued 22 February 1990, J.G. GENG et al., "Rapid Neutrophil Adhesion to Activated Endothelium Mediated by GMP-140", pages 757-760, see entire document.
- Blood, Volume 77, issued 15 April 1991, S. PARMENTIER et al., "Inhibition of Platelet Functions by a Monoclonal Antibody (LYP20) Directed Against a Granule Membrane Glycoprotein (GMP-140/PADGEM)", pages 1734-1739, see entire document.
- Cell, Volume 59, issued 20 October 1989, E. LARSEN et al., "PADGEM Protein: A Receptor that Mediates the Interaction of Activated Platelets with Neutrophils and Monocytes", pages 305-312, see entire document.
- Cell, Volume 56, issued 24 March 1989, G.I. JOHNSTON et al., "Cloning of GMP-140, a Granule Membrane Protein of Platelets and Endothelium: Sequence Similarity to Proteins Involved in Cell Adhesion and Inflammation", pages 1033-1044, see entire document.
- Immunol. Reviews, Volume 114, issued April 1990, T.M. CARLOS et al., "Membrane Proteins Involved in Phagocyte Adherence to Endothelium", pages 5-28, see entire document.

## Description

### BACKGROUND OF THE INVENTION

The present invention relates to compositions and methods for treating inflammation and other pathological conditions mediated by intercellular adhesion. In particular, it relates to inhibition of cellular adhesion using novel immunoglobulins reactive with functional epitopes on a cell surface receptor P-selectin (CD62, granule membrane protein-140 [GMP-140] /platelet activation-dependent granule external membrane [PADGEM]/LECCAM-3, involved in intercellular adhesion.

A specialized cell surface receptor P-selectin, also known as GMP-140, is involved in the recognition of various circulating cells by vascular endothelial cells and platelets. P-selectin is a surface glycoprotein with a lectin-like domain, a region with homology to epidermal growth factor, and a region with homology to complement regulatory proteins (see, McEver *Blood Cells* 16:73-83 [1990]).

The structure of P-selectin is similar to two other vascular cell surface receptors, endothelial leukocyte adhesion molecule (ELAM-1) and lymphocyte homing receptor (LHR). The term "selectin" has been suggested for this general class of receptors, because of their lectin-like domain and the selective nature of their adhesive functions.

These cell surface receptors are expressed on a variety of cells. P-selectin is present on the surface of platelets and endothelial cells in response to a variety of stimuli, where it mediates platelet-leukocyte and endotheliumleukocyte interactions. ELAM-1 is expressed only on endothelial cells and LHR is expressed on a variety of leukocytes in endothelial venules of peripheral lymph nodes.

The role of selectins in inflammation indicates that compounds capable of inhibiting their action may be useful as anti-inflammatory agents. Antibodies to P-selectin have been reported (See e.g., U.S. Patent No. 4,783,330, PCT Application No. US90/06101, PCT Application No. FR90/00565, and McEver et al., J. Biol. Chem. 259:9799-9804 [1984]). In addition, an antibody to P-selectin has been described in presentations at the Scripps Institute, La Jolla California and at a Keystone Colloquium held January 16, 1992. Certain of these antibodies which block P-selectin mediated adhesion are Ca⁺⁺ dependent.

P-selectin is expressed on endothelial cells and on platelets. Expression of P-selectin is inducible and it is not expressed on unactivated endothelial cells or platelets. P-selectin expression does not require de novo synthesis since it is stored in secretory granules (or Weibel-Palade bodies) in both platelets and endothelial cells. Thus, within minutes of activation of either cell type by thrombin, histamine, or phorbol esters or other humoral factors, P-selectin is rapidly redistributed to the surface of the cell where it can adhere to neutrophils, monocytes, and other cells.

There is evidence that P-selectin recognizes Le^{x} as a high affinity ligand (Larsen et al., Cell 63:467-474 [1990]). Additional experiments have been carried out showing that an SLe^{x}-containing oligosaccharide is a more potent inhibitor of P-selectin mediated adhesion than Le^{x} (Polley et al., Proc. Natl. Acad. Sci. USA 88:6224-6228 [1991]). These experiments provide a limited understanding of the ligand specificity of P-selectin in vitro. There are several reports which show that P-selectin binds neutrophils, monocytes and certain carcinoma cells (Bevilacqua et al., Proc. Natl. Acad. Sci. USA 84:9238-9242 [1987]; Geng et al., Nature 343:757-760 [1990]; Larsen et al., Cell 63:467-474 [1990]; and Polley et al., Id.). However, there is little information concerning the precise role of P-selectin in leukocyte recruitment in vivo.

Thus, the therapeutic profile, i.e., whether or not they would be effective in vivo and what diseases they could be used in treating, of prior art anti-P-selectin antibodies is not certain. The prior art lacks evidence showing the effective treatment of inflammatory diseases using antibodies to P-selectin.

J. G. Geng et al (Nature vol. 343, 1990) disclose the use of anti-GMP-140 monoclonal antibodies G1, G2 and S12 and W40 to identify GMP-140 transected Cos cells. The paper shows that antibody G1 blocks adhesion of HL60 cells to GMP-140 transfected Cos cells. Leukocyte adhesion to GMP-140 transfected Cos cells is also inhibited by G2 but not by S12 or W40 suggesting that these two antibodies recognise epitopes not essential for leukocyte binding.

S. Parmentier et al (Blood, Vol. 77, 1991) describe the production of a monoclonal antibody (LYP20) against GMP-140. This antibody specifically bound to thrombin-stimulated platelets compared to control cells and inhibits collagen or thrombin-induced aggregation of washed platelets or platelets in platelet rich plasma. The number of binding sites of LYP20 on thrombin activated platelets was similar to that observed for S12 and KC4. However, the authors concluded that S12 and LYP20 were not directed against the same epitope.

E. Larson et al (Cell, vol. 59, 1989) are concerned with PADGEM protein. The authors show that the protein shares not only structural but also functional homology with ELAM-1 and MEL-14, members of a new family of vascular cell adhesion molecules. The paper discusses the use of anti PADGEM antibodies to see whether they block platelet -HL60 binding.

G. I. Johnston et al (Cell, vol. 56, 1989) are concerned with the cloning of GMP-140. The authors disclose the domains of the protein and conclude that it is similar in structure to ELAM-1 therefore suggesting that GMP-140 belongs to a family of inducible receptors with related structure and function of vascular cells.

T. M. Carlos et al (Immunol. Reviews, vol. 114, 1990) provides a general review paper on phagocyte-endothelial adhesion proteins. The review contains a section on anti-adhesion therapy of inflammatory disorders which discusses the advantages of blocking cell interaction and lists the sort of conditions which could be treated.

International patent application WO 91/06632 concerns the inhibition of PADGEM-mediated cell binding. They disclose the use of soluble agents which competitively inhibit the binding of endothelial cells to monocytes by either binding to PADGEM on the endothelial cells or PADGEM specific ligand on the monocyte.

International patent application WO 92/01718 concerns peptides and ligands for GMP-140 and discloses a peptide having identical sequence to Seq I.D. No. 1 of the present invention. This peptide is from the lectin binding region of GMP-140. There is no teaching in this document as to the production or use of antibodies to GMP-140.

### SUMMARY OF THE INVENTION

The present invention provides a blocking P-selectin antibody that competitively inhibits the binding of an antibody secreted by a cell line designated ATCC accession number HB11041 to P-selectin measured by a competitive inhibition assay. The blocking P-selectin antibodies of the invention bind to P-selectin in the absence of Ca⁺⁺.

The present invention also provides pharmaceutical compositions comprising the antibodies described above in methods for treating inflammatory diseases using these pharmaceutical compositions. Acute lung injury and ischemic reperfusion injury are examples of the inflammatory and thrombotic diseases treatable by the present invention as described more fully below.

### BRIEF DESCRIPTION OF THE DRAWINGS

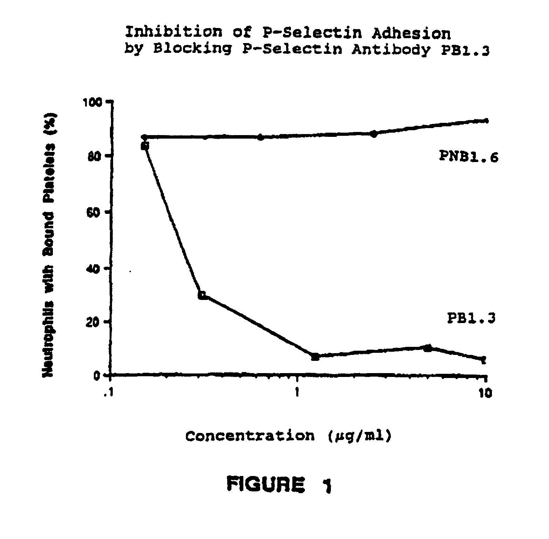

Fig. 1 presents data showing that anti-inflammatory immunoglobulins of the invention inhibit binding of thrombin activated platelets to neutrophils.

Figs. 2A and 2B show that anti-inflammatory immunoglobulins of the invention effectively prevent lung injury induced by infusion of cobra venom factor (CVF) as measured by permeability (Fig. 2A) and hemorrhage (Fig 2B).

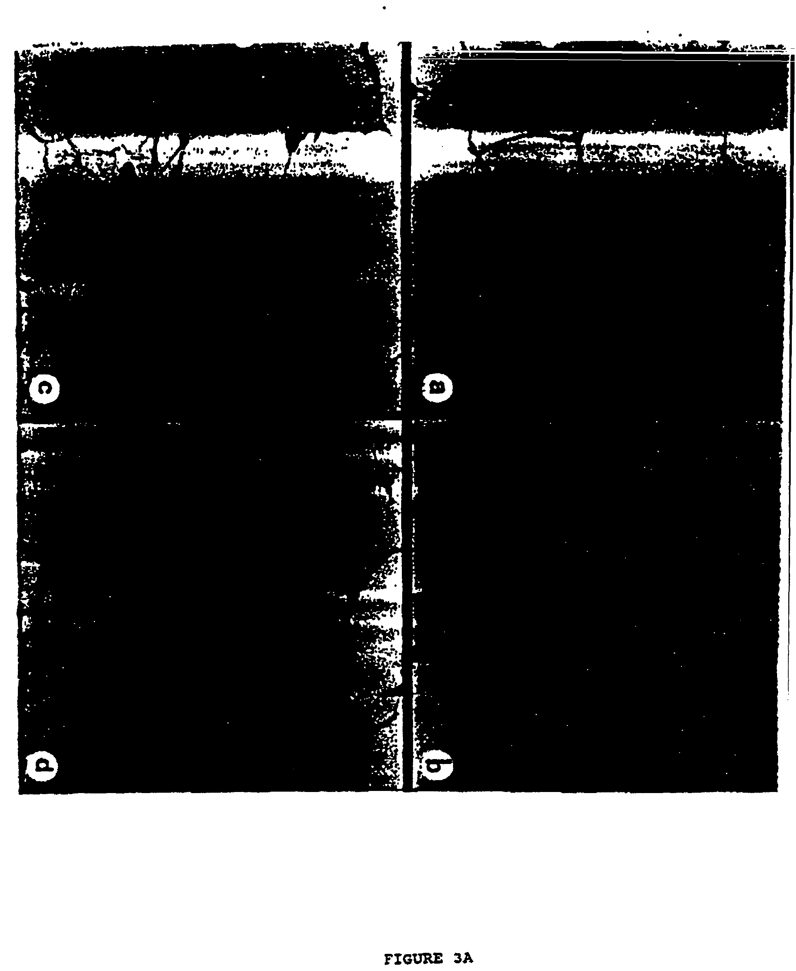

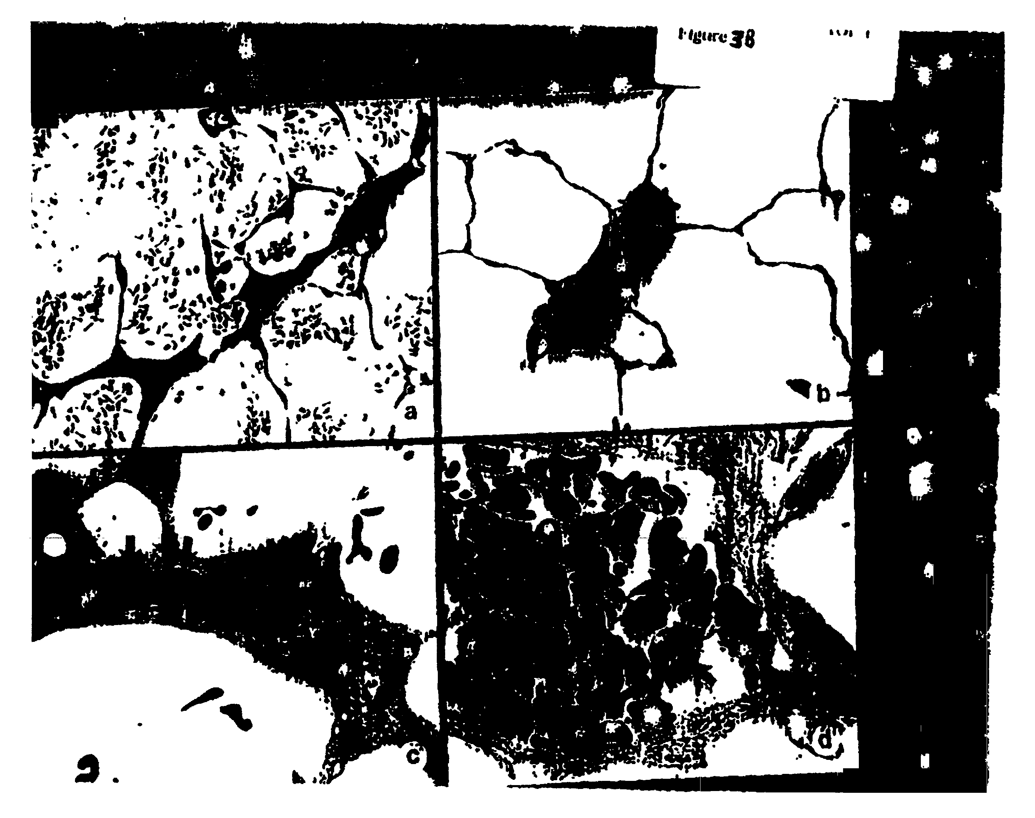

Fig. 3A and 3B show that P-selectin expression content in lungs of animals is upregulated in response to cobra venom infusion visualized by horseradish peroxidase stain of PB1.3 panel (a)-(d) show expression of P-selectin in lung venules at various times after infusion of CVP; time 0 (a); 5, 10 and 15 minutes, panels b, c and d, respectively (Fig. 3A). Fig. 3B are light and transmission electron micrographs of CVF-induced acute lung injury in rats treated with 200 µg non-blocking antibody (PNB1.6) (frames a.c) or with blocking P-selectin antibody (PB1.3) to P-selectin (frames b.d). In animals treated with PNB1.6, vascular injury was indicated by extensive intra-alveolar hemorrhage (frame a), associated with intravascular aggregates of neutrophils (frame c. arrows) in close contrast with endothelial cells. In contrast, in animals treated with PB1.3 intra-alveolar hemorrhage was absent (frame b) and intravascular neutrophils showed little contact with the endothelium (frame d. arrows). (Frames a and b, plastic embedded sections stained with toluidine blue, X160: frames c and d, uranyl acetate, lead citrate stained X2250.)

Fig. 4 shows the binding of the monoclonal antibody PB1.3 to P-selectin: effect of chelating divalent cations with EDTA. In the plot the open symbols (lower trace) is binding in the presence of Ca⁺⁺ and Mg⁺⁺, while the filled symbols (upper trace) is binding in the presence of EDTA. Antibody was diluted shown at 1.6 mg/ml.

Fig. 5 shows the effect of the peptide CQNRYTDLVAIQNKNE (seq. 10 no:1) on the binding of PB1.3 to P-selectin. Filled symbols are in the presence of 0.35 mg/ml peptide, open symbols are in the absence of peptides. Indicated dilutions of PB1.3 were from a 1.6 mg/ml solution.

Figs. 6A, 6B and 6C show the ability of monoclonal antibodies to cross-block binding to P-selectin. Blocking antibodies were present at 50 µg/ml. Dilutions of biotinylated antibodies shown were from 1.6 mg/ml PB1.3, 0.87 mg/ml PNB1.6, and 0.59 mg/ml 84/26.

Fig. 7 shows that the extent of platelet aggregation induced by thrombin is equivalent under control conditions in the absence of antibody and in the presence of increasing concentrations (1-100 µg/ml) of purified PB1.3 antibody.

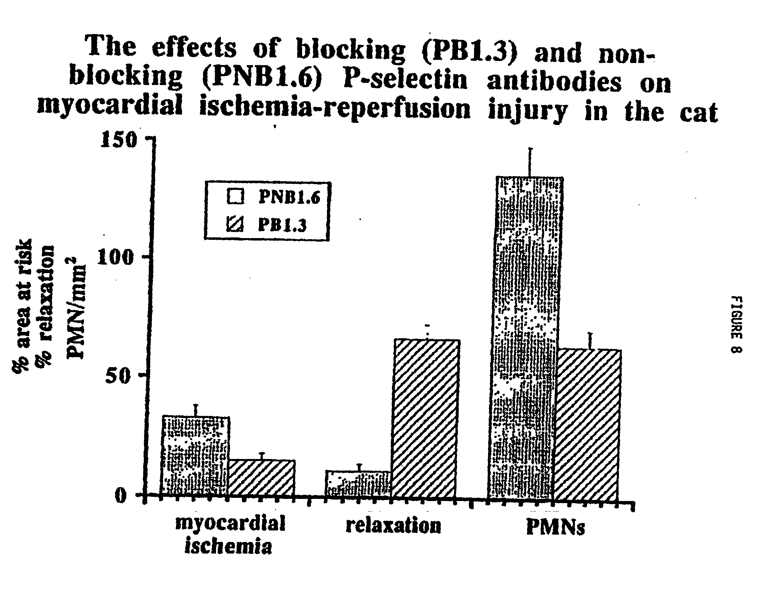

Fig. 8 shows that the extent of myocardial ischemia, expressed as a percentage of the area at risk, is significantly less in animals treated with the blocking anti-P-selectin antibody (PB1.3) than in animals treated with the non-blocking anti-P-selectin antibody. Furthermore, the ability of coronary artery rings to relax in response to acetylcholine is significantly greater in PB1.3 compared with PNB1.6 treated animals. That these phenomena are attributable to a reduction in leukocyte accumulation is suggested by the reduced number of PMNs binding to coronary artery endothelium from PB1.3 treated animals compared with PNB1.6 treated animals.

Fig. 9 (seq ID nos: 23) the DNA sequence and translation of the human immunoglobulin germ-line G-E-A region A: gamma-1 constant region cDNA.

Fig. 10 (seq. ID nos: 4-5) shows the DNA sequence and amino acid sequence of the human kappa immunoglobulin light chain constant domain.

Fig. 11 (seq. ID nos: 6-7) shows the sequence and translation of PB1.3 heavy chain signal peptide and variable region.

Fig. 12 (seq. ID nos:8-9) shows the sequence and translation of PB1.3 light chain signal peptide and variable region.

Fig. 13 (seq. ID nos:10-11) shows the sequence and translation of PB1.3/Humanized heavy chain-A signal peptide and variable region.

Fig. 14 (seq ID nos:12-13) shows the sequence and translation of PB1.3/Humanized heavy chain-B signal peptide and variable region.

Fig. 15 (seq. ID nos:16-17) shows the sequence and translation of PB1.3/Humanized light chain-A signal peptide and variable region.

Fig. 16 (seq. ID nos: 18-19) shows the sequence and translation of PB1.3/Humanized light chain-B signal peptide and variable region.

Fig. 17 (seq. ID nos: 14-15) shows the sequence and translation of PB1.3/Humanized heavy chain-C signal peptide and variable region.

Fig. 18 shows the changes in ear volume following tissue injury after administration of PB1.3 or a control murine monoclonal antibody.

Fig. 19 shows the development of necrosis following ischemia and reperfusion of the rabbit ear after administration of PB1.3 and a control murine monoclonal antibody.

Fig. 20 shows the inhibition of mast cell induced intravascular leukocyte accumulation after administration of PB1.3.

### DESCRIPTION OF THE PREFERRED EMBODIMENT

This invention concerns compositions and methods for inhibiting inflammatory and other diseases in which P-selectin is involved. Specifically, the invention provides immunoglobulins which have the ability to inhibit selectin-mediated adhesion of the cells in vivo. The immunoglobulins of the present invention selectively bind functional epitopes on P-selectin and effectively block adhesion of leukocytes to the vascular endothelium. The present invention also provides diagnostic and therapeutic uses for these immunoglobulins.

"Blocking P-selectin antibodies" shall mean the anti-P-selectin immunoglobulins of this invention which inhibit the binding of neutrophils to activated platelets and/or to activated vascular endothelium in vivo. Blocking P-selectin antibodies are suitable for modification using the multitude of techniques available to those skilled in the art for production and manipulation of various immunoglobulin molecules. Blocking P-selectin antibodies are proteins consisting of one or more polypeptides substantially encoded by immunoglobulin genes. The recognized immunoglobulin genes include the kappa, lambda, alpha, gamma, delta, epsilon and mu constant region genes, as well as the myriad immunoglobulin variable region genes. For a discussion of immunoglobulin forms, see e.g., Fundamental Immunology, 2d Ed. W.E. Paul ed., Raven Press NY [1989], Huston et al., Proc. Natl. Acad. Sci. U.S.A. 85:5879-5883 [1988], Bird et al., Science 242:423-426 [1988], and Hunkapiller and Hood, Nature 323:15-16 [1986].

As used herein, "immunoglobulin," "antibody" or "antibody peptide(s)" refers to antibodies, monoclonal antibodies, to an entire immunoglobulin or antibody or any functional fragment of an immunoglobulin molecule which binds to the functional epitope of P-selectin to which the blocking P-selectin antibodies of this invention bind. Examples of such peptides include complete antibody molecules, antibody fragments, such as Fab, F(ab')₂, complementarity determining regions (CDRs), V_{L} (light chain variable region), V_{H} (heavy chain variable region), and any combination of those or any other functional portion of an antibody peptide.

An F(ab')₂ fragment lacks the C-terminal portion of the heavy chain constant region, and has a molecular weight of approximately 110 kD. It retains the two antigen binding sites and the interchain disulfide bonds in the hinge region, but it does not have the effector functions of an intact IgG molecule. An F(ab')₂ fragment may be obtained from an IgG molecule by proteolytic digestion with pepsin at pH 3.0-3.5 using standard methods such as those described in Harlow and Lane, Antibodies: A Laboratory Manual, Cold Spring Harbor Pubs., N.Y. [1988].

An Fab fragment comprises a light chain and the N-terminus portion of the heavy chain to which it is linked by disulfide bonds. It has a molecular weight of approximately 50 kD and contains a single antigen binding site. Fab fragments may be obtained from F(ab')₂ fragments by limited reduction, or from whole antibody by digestion with papain in the presence of reducing agents. (See, Harlow and Lane, supra.)

A multitude of techniques available to those skilled in the art for production and manipulation of various immunoglobulin molecules can be readily applied to produce antibodies for use in the present invention. Antibodies which bind to p-selectin may be produced by a variety of means. The production of non-human monoclonal antibodies, e.g., murine, lagomorpha, equine, etc., is well known and may be accomplished by, for example, immunizing the animal with a preparation containing cells bearing P-selectin (e.g., thrombin-activated platelets) or isolated P-selectin molecules. Antibody-producing cells obtained from the immunized animals are immortalized and screened, or screened first for the production of antibody which binds to P-selectin and then immortalized. For a discussion of general procedures of monoclonal antibody production see Harlow and Lane, supra.

Less preferred forms of immunoglobulins may be produced by methods well-known to those skilled in the art. An example is chromatographic purification of polyclonal sera to produce substantially monospecific antibody populations.

A preferred monoclonal antibody for use in this invention is produced in the hybridoma cell line deposited with the American Type Culture Collection in Rockville, Maryland (ATCC) under the Budapest Treaty and given the Accession No. HB11041. The production of this monoclonal antibody is described in Example 2, below.

The generation of human monoclonal antibodies to a human antigen is also known in the art. Generation of such human monoclonal antibodies may be difficult with conventional techniques. Thus, it may be desirable to isolate DNA sequences which encode blocking P-selectin human monoclonal antibody (or portions thereof) by screening a DNA library from human B cells according to the general protocol outlined by Huse et al., Science 246:1275-1281 [1989]. The sequences which encode the antibody (or binding fragment) of the desired specificity are then cloned and amplified. Alternatively, one may transfer the antigen binding regions of non-human antibodies, e.g., the F(ab')₂ or hypervariable regions, to human constant regions (Fc) or framework regions by recombinant DNA techniques to produce substantially human molecules. Such methods are generally known in the art and are described below.

Thus, the invention also provides synthetic or recombinant blocking P-selectin antibodies, including chimeric immunoglobulins, humanized antibodies or hybrid antibodies or derivatives of any of those. Chimeric immunoglobulins are typically the product of chimeric DNA, which is recombinant DNA containing genetic material from more than one mammalian species.

"Chimeric immunoglobulins" or "chimeric antibodies" refer to those antibodies or antibody peptides wherein one portion of the peptide has an amino acid sequence that is derived from, or is homologous to, a corresponding sequence in a blocking P-selectin antibody of this invention or peptide derived from a first gene source, while the remaining segment of the chain(s) is homologous to corresponding sequences of another gene source. For example, a chimeric blocking P-selectin antibody peptide may comprise an antibody heavy chain with a murine variable region and a human constant region. The two gene sources will typically involve two species, but will occasionally involve different sources from one species.

Chimeric blocking P-selectin antibodies or peptides are typically produced using recombinant molecular and/or cellular techniques. Typically, chimeric antibodies have variable regions of both light and heavy chains that mimic the variable regions of antibodies derived from one mammalian species, while the constant portions are homologous to the sequences in antibodies derived from a second, different mammalian species. Methods for production of such antibodies are well known and are described in, for example, U.S. 4,816,397, EP publications 173,494 and 239,400.

The definition of a chimeric immunoglobulin, however, is not limited to this example. A chimeric antibody is any antibody in which either or both of the heavy or light chains are composed of combinations of sequences mimicking the sequences in antibodies of different sources, whether these sources be differing classes, differing antigen responses, or differing species of origin, and whether or not the fusion point is at the variable/constant boundary.

The term "humanized" or "substantially human immunoglobulin" refers to a blocking immunoglobulin comprising a substantially human framework region and a constant region that corresponds substantially to a human immunoglobulin constant region. Hence, most parts of a humanized immunoglobulin, except possibly the CDRs, are substantially homologous to corresponding parts of one or more native human immunoglobulin sequences.

"Hybrid antibody" refers to an antibody wherein each chain is separately homologous with reference to a mammalian antibody chain, but the combination represents a novel assembly so that two different antigens are recognized by the antibody. In hybrid antibodies, one heavy and light chain pair is homologous to a pair found in an antibody raised against another epitope. This results in the property of multi-functional valency, i.e., ability to bind at least two different epitopes simultaneously, wherein at least one epitope is the epitope to which the blocking P-selectin antibody binds. Such hybrids may, of course, also be formed using chimeric chains.

Thus, in one aspect, the present invention is directed to recombinant DNA segments encoding the heavy and/or light chain variable or hypervariable regions from blocking P-selectin immunoglobulin genes. The DNA segments encoding these regions will typically be joined to DNA sequences encoding appropriate constant regions, such as human gamma heavy chain regions or human kappa light chain regions. One of skill will recognize that, due to codon degeneracy and non-critical amino-acid substitutions, other DNA sequences can be readily substituted for those sequences, as detailed below.

The DNA segments will typically further include an expression control DNA sequence operably linked to the chimeric antibody coding sequences, including naturally-associated or heterologous promoter regions. Preferably, the expression control sequences will be eukaryotic promoter systems in vectors capable of transforming or transfecting eukaryotic host cells. Once the vector has been incorporated into the appropriate host, the host is maintained under conditions suitable for high level expression of the nucleotide sequences, and, as desired, the collection and purification of the encoded immunoglobulins may follow.

It is well known that native forms of "mature" immunoglobulins will vary somewhat in terms of length by deletions, substitutions, insertions or additions of one or more amino acids in the sequences. Thus, both the variable and constant regions are subject to substantial natural modification, yet are "substantially identical" or "substantially homologous" and still capable of retaining their respective activities. Suitable source cells for the DNA sequences and host cells for expression and secretion can be obtained from a number of sources, such as the American Type Culture Collection ("Catalogue of Cell Lines and Hybridomas," Fifth edition [1985] Rockville, Maryland, U.S.A.

In addition to these naturally-occurring forms of immunoglobulin chains, substantially identical modified heavy and light chains can be readily designed and manufactured utilizing various recombinant DNA techniques well known to those skilled in the art. For example, the chains can vary from the naturally-occurring sequence at the primary structure level by several amino acid substitutions, terminal and intermediate additions and deletions, and the like. Alternatively, polypeptide fragments comprising only a portion (usually at least about 60-80%, typically 90-95%) of the primary structure may be produced, which fragments possess one or more immunoglobulin activities (e.g., complement fixation activity), while exhibiting lower immunogenicity. In particular, it is noted that like many genes, the immunoglobulin-related genes contain separate functional regions, each having one or more distinct biological activities. These may be fused to functional regions from other genes (e.g., enzymes) to produce fusion proteins (e.g., immunotoxins) having novel properties. In general, modifications of the genes may be readily accomplished by a variety of well-known techniques, such as site-directed mutagenesis (see, Gillman and Smith, Gene, 8:81-97 [1979] and Roberts, et al., Nature, 328:731-734 [1987]).

As stated previously, the DNA sequences will be expressed in hosts after the sequences have been operably linked to an expression control sequence. These expression vectors are typically replicable in the host organisms either as episomes or as an integral part of the host chromosomal DNA. Commonly, expression vectors will contain selection markers, e.g., tetracycline or neomycin, to permit detection of those cells transformed with the desired DNA sequences (see, e.g., U.S. Patent 4,704,362).

E. coli is one prokaryotic host particularly useful for cloning the DNA sequences and constructing the various vectors of the present invention. For example, E. coli K12 strain 294 (ATCC No. 31446) is particularly useful. Other microbial strains which may be used include E. coli B and E. coli X1776 (ATCC no. 31537). These examples are illustrative rather than limiting.

Prokaryotes are also used for expression. The aforementioned strains, as well as E. coli W3110 (F⁻ λ⁻, prototrophic, ATCC No. 27325), bacilli such as Bacillus Subtilis, and other enterobacteriaceae such as Salmonella typhimurium or Serratia marcescans, and various pseudomonas species may be used.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, E. coli is typically transformed using pBR322, a plasmid derived from an E. coli species (Bolivar et al., Gene 2, 95 [1977]). pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA construction.

Promoters suitable for use with prokaryotic hosts illustratively include the β-lactamase and lactose promoter systems (Chang et al., Nature, 275, 615 [1976]; and Goeddel et al., Nature, 281, 544 [1979]), alkaline phosphatase, the tryptophan (trp) promoter system (Goeddel, Nucl. Acids Res. 8:4057 [1980]) and hybrid promoters such as the tac promoter (de Boer, Proc. Natl. Acad. Sci. USA 80:21-25 [1983]). However, other functional bacterial promoters are suitable. Their nucleotide sequences are generally known, thereby enabling a skilled worker operably to ligate them to DNA encoding sialyltransferase (Siebenlist et al., Cell 2: June; 20: 269-281 [1980]) using linkers or adaptors to supply any required restriction sites. Promoters for use in bacterial systems also will contain a Shine-Dalgarno (S.D.) sequence operably linked to the DNA encoding sialyltransferase.

In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (Stinchomb et al., Nature 282, 39 [1979]; Kingsman et al., Gene 7, 141 [1979]; Tschemper et al., Gene 10, 157 [1980]) is commonly used. This plasmid already contains the trpl gene which provides a selection marker for a mutant strain of yeast lacking the ability to grow in tryptophan, for example ATCC no. 44076 or PEP4-1 (Jones, Genetics 85, 12 [1977]). The presence of the trpl lesion as a characteristic of the yeast host cell genome then provides an effective environment for detecting transformation by growth in the absence of tryptophan.

Suitable promoting sequences for use with yeast hosts include the promoters for 3-phosphoglycerate kinase (Hitzeman et al., J. Biol. Chem. 255, 2073 [1980]) or other glycolytic enzymes (Hess et al., J. Adv. Enzyme Reg. 7, 149 [1968]; and Holland, Biochemistry 17, 4900 [1978]), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., European Patent Publication No. 73,657A. Yeast enhancers also are advantageously used with yeast promoters.

In addition to microorganisms, mammalian tissue cell culture may also be used to produce the polypeptides of the present invention (see, Kriegler, Gene Transfer and Expression: A Laboratory Manual, W.H. Freeman, N.Y., [1990]). Eukaryotic cells are preferred, because a number of suitable host cell lines capable of secreting intact immunoglobulins have been developed in the art, and include the CHO cell lines, various COS cell lines, HeLa cells, myeloma cell lines, transformed B-cells, and hybridomas.

"Control region" refers to specific sequences at the 5' and 3' ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription of many genes is a CXCAAT region where X may be any nucleotide. At the 3' end of most eukaryotic genes is an AATAAA sequence which may be the signal for addition of the poly A tail to the 3' end of the transcribed mRNA.

Preferred promoters controlling transcription from vectors in mammalian host cells may be obtained from various sources, for example, the genomes of viruses such as: polyoma, Simian Virus 40 (SV40), adenovirus, retroviruses, hepatitis-β virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273, 113 ([1978]). The immediate early promoter of the human cytomegalovirus is conveniently obtained as a HindIII restriction fragment (Greenaway, P.J. et al., Gene 18 355-360 [1982]). Of course, promoters from the host cell or related species also are useful herein.

Transcription of a DNA encoding the immunoglobulin based proteins by higher eukaryotes is increased by inserting an enhancer sequence into the vector. Enhancers are cis-acting elements of DNA, usually from about 10-300bp, that act on a promoter to increase its transcription. Enhancers are relatively orientation and position independent having been found 5' (Laimins, et al., Proc. Natl. Acad. Sci. USA 78, 993 [1981]) and 3' (Lusky, et al., Mol. Cell Bio. 3, 1108 [1983]) to the transcription unit, within an intron (Banerji, et al., Cell 33, 729 [1983]) as well as within the coding sequence itself (Osborne, et al., Mol. Cell Bio. 4, 1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding P-selectin antibody. The 3' untranslated regions also include transcription termination sites.

Expression vectors may contain a selection gene, also termed a selectable marker. Examples of suitable selectable markers for mammalian cells are dihydrofolate reductase (DHFR), ornithine decarboxylase, multiple drug resistance biochemical marker, adenosine deaminase, asparagine synthetase, glutamine synthetase, thymidine kinase or neomycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are CHO DHFR- cells and mouse LTK- cells. These cells lack the ability to grow without the addition of such nutrients as thymidine or hypoxanthine. Because those cells lack certain genes necessary for a complete nucleotide synthesis pathway, they cannot survive unless the missing nucleotide synthesis pathway, are provided in a supplemented media. An alternative to supplementing the media is to introduce an intact DHFR or TK gene into cells lacking the respective genes, thus altering their growth requirement. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin (Southern and Berg, J. Molec. Appl. Genet, 1, 327 [1982]), mycophenolic acid (Mulligan and Berg, science 209, 1422 [1980]) or hygromycin (Sugden et al., Mol. Cell Biol. 5, 410-413 [1985]). The three examples given above employ bacterial genes under eukaryotic control to convey resistance to the appropriate drug G418 or neomycin (genticin), xgpt (mycophenolic acid) or hygromycin, respectively.

"Amplification" refers to the increase or replication of an isolated region within a cell's chromosomal DNA. Amplification is achieved using a selection agent e.g. methotrexate (MTX) which inactivates DHFR. Amplification or the accumulation of multiple copies of the DHFR gene results in greater amounts of DHFR being produced in the face of greater amounts of MTX. Amplification pressure is applied notwithstanding the presence of endogenous DHFR, by adding ever greater amounts of MTX to the media. Amplification of a desired gene can be achieved by cotransfecting a mammalian host cell with a plasmid having a DNA encoding a desired protein and the DHFR or amplification gene by cointegration is referred to as coamplification. One ensures that the cell requires more DHFR, which requirement is met by replication of the selection gene, by selecting only for cells that can grow in the presence of ever-greater MTX concentration. So long as the gene encoding a desired heterologous protein has cointegrated with the selection gene, replication of this gene often gives rise to replication of the gene encoding the desired protein. The result is that increased copies of the gene, i.e. an amplified gene, encoding the desired heterologous protein express more of the desired heterologous protein.

Preferred suitable host cells for expressing the vectors of this invention encoding the immunoglobulin based proteins in higher eukaryotes include: monkey kidney CV1 line transformed by SV40 (COS-7, ATCC CRL 1651); human embryonic kidney line (293) (Graham et al., J. Gen. Virol. 36, 59 [1977]); baby hamster kidney cells (BHK, ATCC CCL 10); chinese hamster ovary-cells-DHFR (CHO, Urlaub and Chasin, Proc. Natl. Acad. Sci. (USA) 77, 4216 [1980]); mouse sertoli cells (TM4, Mather, Biol. Reprod. 23, 243-251 [1980]); monkey kidney cells (CV1 ATCC CCL 70); african green monkey kidney cells (VERO-76, ATCC CRL 1587) ; human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); and, TRI cells (Mather, et al., Annals N.Y. Acad. Sci. 383, 44-46 [1982]); baculovirus cells.

"Transformation" means introducing DNA into an organism so that the DNA is replicable, either as an extrachromosomal element or by chromosomal integration. Unless indicated otherwise, the method used herein for transformation of the host cells is the method of Graham and Van der Eb, Virology 52, 456-457 [1973]. However, other methods for introducing DNA into cells such as by nuclear ingestion or by protoplast fusion may also be used. If prokaryotic cells or cells which contain substantial cell wall constructions are used, the preferred method of transfection is calcium treatment using calcium chloride as described by Cohen et al., Proc. Natl. Acad. Sci. USA 69, 2110 [1972].

For analysis to confirm correct sequences in plasmids constructed, the ligation mixtures are used to transform E coli K12 strain 294 (ATCC 31446) and successful transformants selected by ampicillin or tetracycline resistance where appropriate. Plasmids from the transformants are prepared, analyzed by restriction and/or sequenced by the method of Messing et al., Nucleic Acids Res. 9, 309 [1981] or by the method of Maxam et al., Methods in Enzymology 65, 449 [1980].

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinary skilled artisan.

"Transfection" refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

The vectors containing the DNA segments of interest (e.g., the heavy and light chain encoding sequences and expression control sequences) can be transferred into the host cell by well-known methods, which vary depending on the type of cellular host. For example, calcium chloride transfection is commonly utilized for prokaryotic cells, whereas calcium phosphate treatment may be used for other cellular hosts. See, generally, Sambrook et al., Molecular Cloning: A Laboratory Manual, 2nd Ed., Cold Spring Harbor Press, [1989].

Once expressed, the whole chimeric antibodies, their dimers, or individual light and heavy chains of the present invention can be purified according to standard procedures of the art, including ammonium sulfate precipitation, fraction column chromatography, gel electrophoresis and the like.

For this invention, a blocking P-selectin immunoglobulin is specific for, or reactive with a functional epitope on P-selectin molecule if the immunoglobulin binds the functional epitope on P-selectin defined by the blocking P-selectin antibody of this invention as measured or determined by standard antibody-antigen assays, for example, competitive binding assays, saturation assays, or standard immunoassays such as ELISA or RIA. Binding to this functional epitope is not inhibited by the peptide CQNRYTDLVAIQNKNE. Ca⁺⁺ is not necessary for binding. This definition of specificity applies to single heavy and/or light chains, CDRs, fusion proteins or fragments of heavy and/or light chains, that are also specific for P-selectin if they bind P-selectin alone or if, when properly incorporated into immunoglobulin conformation with complementary variable regions and constant regions as appropriate, are then capable of binding P-selectin.

The immunoglobulins of the present invention preferably have sufficient affinity to assure binding to the functional epitope of P-selectin. Binding affinity is typically represented by the affinity constant (Kₐ) for equilibrium concentrations of associated and disassociated configurations, i.e., Kₐ = [A-B]/[A][B] where [A], [B], and [A-B] are the concentrations at equilibrium of the antibody (A), antigen (B) and antigen-antibody complex (A-B), respectively. One of skill will recognize, however, that binding affinity between two molecules will be influenced by a number of factors such as temperature, pH, ionic strength, and the like.

Based on the activators of P-selectin and ELAM-1, it has been postulated that their expression represents inflammatory and hemostatic responses to tissue injury. However, multiple adhesion proteins and their ligands are required for leukocyte adhesion to and extravasation across endothelial cells. It was not until this invention that the specific adhesion receptor P-selectin was shown in vivo to be critical to a response to tissue injury. The blocking P-selectin antibodies of the present invention comprise immunoglobulins which selectively bind a functional epitope on P-selectin on cells associated with a response to tissue injury and inflammation.

Inflammatory and thrombotic conditions treatable with the present invention include, e.g., post-ischemic leukocyte-mediated tissue damage (reperfusion injury) such as traumatic shock, stroke, myocardial infarction, acute transplantation rejection, frost-bite injury, compartment syndrome, and pathophysiologic conditions associated with cardio-pulmonary bypass, acute leukocyte-mediated lung injury (e.g., adult respiratory distress syndrome), septic shock, wound associated sepsis secondary to viral infection such as herpes simplex virus, IgE mediated allergic reactions such as acute phase asthmatic disease, and chronic inflammatory conditions, including rheumatoid arthritis, atopic dermatitis and psoriasis. In addition, tumor metastasis can be prevented by inhibiting the adhesion of circulating cancer cells. Examples include carcinoma of the colon and melanoma.

One major component of acute allergic conditions, including asthma, is the degranulation of mast cells following challenge of subjects with antigens to which they are specifically sensitized. The consequences of mast cell degranulation include a bronchoconstrictor response and also an inflammatory response characterized in part by leukocyte accumulation. Histamine, which is contained together with other inflammatory mediators in mast cells, can induce the expression of P-selectin on vascular endothelial cells, indicating that degranulation of mast cells may also result in P-selectin expression and subsequent leukocyte accumulation. Because mast cell degranulation is a key element in the pathogenesis of allergic conditions, administration of antibodies to P-selectin will be useful for the treatment of human allergic conditions,

The blocking P-selectin antibodies and pharmaceutical compositions of this invention are particularly useful for parenteral administration, i.e., subcutaneously, intramuscularly or intravenously. A number of new drug delivery approaches are being developed, the pharmaceutical compositions of the present invention are suitable for administration using these new methods, as well. See, Langer, Science, 249:1527-1533 [1990].

The blocking P-selectin antibodies can be directly or indirectly coupled to the chemotherapeutic agent. The coupling, which may be performed by means generally known in the art, should not substantially inhibit the ability of the immunoglobulin to bind the receptor nor should it substantially reduce the activity of the chemotherapeutic agent. A variety of chemotherapeutics can be coupled for targeting. For example, anti-inflammatory agents which may be coupled include immunomodulators, platelet activating factor (PAF) antagonists, cyclooxygenase inhibitors, lipoxygenase inhibitors, and leukotriene antagonists. Some preferred moieties include cyclosporin A, indomethacin, naproxen, FK-506, mycophenolic acid, and the like. similarly, anti-oxidants, e.g., superoxide dismutase, are useful in treating reperfusion injury. Likewise, anticancer agents, such as daunomycin, doxorubicin, vinblastine, bleomycin, and the like can be targeted.

The P-selectin targeting may also be accomplished via amphipaths, or dual character molecules (polar:nonpolar) which exist as aggregates in aqueous solution. Amphipaths include nonpolar lipids, polar lipids, mono- and diglycerides, sulfatides, lysolecithin, phospholipids, saponin, bile acids and salts. These molecules can exist as emulsions and foams, micelles, insoluble monolayers, liquid crystals, phospholipid dispersions and lamellar layers. These are generically referred to herein as liposomes. In these preparations, the drug to be delivered is incorporated as part of a liposome in which an anti-P-selectin immunoglobulin is embedded. In this embodiment, the immunoglobulin need not bind a functional epitope on the P-selectin molecule, so long as the immunoglobulin effectively targets the liposome to P-selectin molecules. When the liposomes are brought into proximity of the affected cells, they deliver the selected therapeutic compositions.

A variety of methods are available for preparing liposomes, as described in, *e*.*g*., Szoka et al., Ann. Rev. Biophys. Bioeng. 9:467 [1980], U.S. Pat. Nos. 4,235,871, 4,501,728 and 4,837,028. Targeting of liposomes using a variety of targeting agents (e.g., ligands, receptors and monoclonal antibodies) is well known in the art. (see, e.g., U.S. Patent Nos. 4,957,773 and 4,603,044. Standard methods for coupling targeting agents to liposomes can be used. Antibody targeted liposomes can be constructed using, for instance, liposomes which incorporate protein A (see, Renneisen, et al., J. Biol. Chem., 265:16337-16342 [1990] and Leonetti et al., Proc. Natl. Acad. Sci. (USA) 87:2448-2451 [1990].

As discussed above, the pharmaceutical compositions (comprising targeted liposomes or free antibodies) are particularly suitable for parenteral administration. The composition will commonly comprise a solution of the antibody or a cocktail thereof dissolved in an acceptable carrier, preferably an aqueous carrier. A variety of aqueous carriers can be used, e.g., water, buffered water, 0.4% saline, 0.3% glycine and the like. These solutions are sterile and generally free of particulate matter. These compositions may be sterilized by conventional, well known sterilization techniques. The compositions may contain pharmaceutically acceptable auxiliary substances as required to approximate physiological conditions such as pH-adjusting and buffering agents, tonicity adjusting agents and the like, for example sodium acetate, sodium chloride, potassium chloride, calcium chloride, sodium lactate, etc. The concentration of antibody in these formulations can vary widely, i.e., from less than about 0.5%, usually at or at least about 1% to as much as 15 or 20% by weight and will be selected primarily based on fluid volumes, viscosities, etc., in accordance with the particular mode of administration selected. Actual methods for preparing parenterally administrable compositions will be known or apparent to those skilled in the art and are described in more detail in, for example, Remington's Pharmaceutical Sciences, 17th Ed., Mack Publishing company, Easton, Pennsylvania [1985].

The antibodies of this invention can be lyophilized for storage and reconstituted in a suitable carrier prior to use. This technique has been shown to be effective with conventional immunoglobulins and art-known lyophilization and reconstitution techniques can be employed. It will be appreciated by those skilled in the art that lyophilization and reconstitution can lead to varying degrees of antibody activity loss (e.g., with conventional immunoglobulins, IgM antibodies tend to have greater activity loss than IgG antibodies) and that use levels may have to be adjusted to compensate.

The compositions containing the present antibodies or a cocktail thereof can be administered for the prophylactic and/or therapeutic treatments. In therapeutic application, compositions are administered to a patient in an amount sufficient to cure or at least partially arrest the infection and its complications. An amount adequate to accomplish this is defined as a "therapeutically effective dose." Amounts effective for this use will depend upon the severity of the disease and the general state of the patient's own immune system, but generally range from about .05 mg/kg body weight to about 5 mg/kg body weight, preferably between about .2 mg/kg body weight to about 1.5 mg/kg body weight.

It must be kept in mind that the materials of this invention may generally be employed in serious disease states, that is life-threatening or potentially life-threatening situations. In such cases, in view of the minimization of extraneous substances and the lower probability of "foreign substance" rejections which are achieved by human chimeric antibody forms of this invention, it is possible and may be felt desirable by the treating physician to administer substantial excesses of these antibodies.

In prophylactic applications, compositions containing the present antibodies or a cocktail thereof are administered to a patient not already in a disease state to enhance the patient's resistance. Such an amount is defined to be a "prophylactically effective dose." In this use, the precise amounts again depend upon the patient's state of health and general level of immunity, but generally in the ranges described above.

Single or multiple administrations of the compositions can be carried out with dose levels and pattern being selected by the treating physician. In any event, the pharmaceutical formulations should provide a quantity of the immunoglobulins of this invention sufficient to effectively treat the patient.

The antibodies of the present invention may also be used for diagnostic purposes, such as identifying areas of inflammation. For diagnostic purposes, the antibodies may either be labeled or unlabeled. Unlabeled antibodies can be used in combination with other labeled antibodies (second antibodies) that are reactive with the antibody, such as antibodies specific for the particular immunoglobulin constant region. Alternatively, the antibodies can be directly labeled. A wide variety of labels may be employed, such as radionuclides, fluorescers, enzymes, enzyme substrates, enzyme cofactors, enzyme inhibitors, ligands (particularly haptens), etc. Numerous types of immunoassays are available and are well known to those skilled in the art.

In diagnostic applications, compositions containing the immunoglobulins or a cocktail thereof, are administered to a patient suspected of having an inflammatory disease state. Alternatively, the efficacy of a particular treatment can be monitored. An amount sufficient to accomplish this is defined to be a "diagnostically effective dose." In this use, the precise amounts will depend upon the patient's state of health and the like.

Kits can also be supplied for use with the subject antibodies. Thus, the subject antibody composition of the present invention may be provided, usually in a lyophilized form in a container, either alone or in conjunction with additional antibodies specific for the desired cell type. The antibodies, which may be conjugated to a label or toxin, or unconjugated, are included in the kits with buffers, such as Tris, phosphate, carbonate, etc., stabilizers, biocides, inert proteins, e.g., serum albumin, or the like, and a set of instructions for use. Generally, these materials will be present in less than about 5% wt. based on the amount of active antibody, and usually present in total amount of at least about 0.001% wt. based again on the antibody concentration. Frequently, it will be desirable to include an inert extender or excipient to dilute the active ingredients, where the excipient may be present in from about 1 to 99% wt. of the total composition. Where a second antibody capable of binding to the chimeric antibody is employed in an assay, this will usually be present in a separate vial. The second antibody is typically conjugated to a label and formulated in an analogous manner with the antibody formulations described above.

A "functional epitope" is an antigenic site bound by blocking P-selectin antibody which binding effectively blocks adhesion of leukocytes to vascular endothelium associated with an inflammatory and/or thrombotic condition.

As used herein "substantial inhibition" is at least about 50% inhibition, preferably about 60% to about 80%, and more usually about greater than 85% or more (as measured in an in vitro competitive binding assay).

An inmunoglobulin is "reactive with" or "binds to" an antigen if it interacts with the antigen. Typically, the binding interactions between ligand or peptide and receptor or antigen involve reversible noncovalent associations such as electrostatic attraction, Van der Waals forces and hydrogen bonds. This interaction is analogous to a chemical reaction in which two reactants come together to form a product. In the case of the immunoglobulin-antigen interaction, the product of the interaction is an antigen-immunoglobulin complex.

A "competitive binding assay" is one which measures the ability of an immunoglobulin to inhibit binding of a blocking P-selectin immunoglobulin (e.g., one secreted by ATCC Accession No. HB11041) to an antigenic determinant on a P-selectin molecule. Numerous types of competitive binding assays are known for example: solid phase direct or indirect radioimmunoassay (RIA), solid phase direct or indirect enzyme immunoassay (EIA), sandwich competition assay (see, Stahli et al., Methods in Enzymology 9: 242-253 [1983]); solid phase direct biotin-avidin EIA (see, Kirkland et al., J. Immunol. 137:3614-3619 [1986]); solid phase direct labeled assay, solid phase direct labeled sandwich assay (see, Harlow and Lane, "Antibodies, A Laboratory Manual," Cold spring Harbor [1988]); solid phase direct label RIA using I-125 label (see, Morel et al., Molec. Immunol. 25(1):7-15 [1988]); solid phase direct biotin-avidin EIA (Cheung et al., Virology 176:546-552 [1990]); and direct labeled RIA (Moldenhauer et al., Scand. J. Immunol. 32:77-82 [1990]). Typically, such an assay involves the use of purified P-selectin or cells bearing P-selectin bound to a solid surface and a labeled immunoglobulin known to be reactive with P-selectin. The immunoglobulin to be tested is added and competitive inhibition is measured by determining the amount of label bound to the solid surface in the presence of the test immunoglobulin. Standard procedures for monoclonal antibody assays, such as ELISA, may be used. An example of a suitable competitive binding assay is presented in Example 8, below.

The following examples are offered by way of illustration, not by limitation.

### Example 1

### Preparation of Reagents

This example describes preparation or isolation of reagents used for the immunizations in Examples 2 and 3 and the ELISAs in Example 4.

### A. "OUTDATED PLATELETS"

A preparation-of platelets which was purified from "outdated platelets" obtained from the San Diego Blood Bank. The method used was a modification of the method described in Moore et al., J. Cell. Biol. 112:491-499 [1991]. Briefly, 25 units of outdated platelet-rich plasma were centrifuged twice for 10 min at 1200rpm (300xg) to remove contaminating non-platelet blood cells. The purified platelet preparation was then washed three times with a buffer containing 0.1M NaCl, 20mM Tris and 5mM Benzamidine. It also contained 3.8% sodium citrate. The pH was adjusted to 7.5 with 1N HCl. The purified platelets were stored at 70°C and were washed once in PBS before injection.

### B. PURIFIED P-SELECTIN

Fractionation of Platelets: Washed outdated platelets were fractionated essentially according to Moore et al., 1991, supra. 25 units washed platelets were made 100 µM in leupeptin and 1 mM in 4-(2-aminoethyl)-benzenesulfonylfluoride (AEBSF), freeze-thawed three times in dry ice/methanol, and homogenized ten strokes with a Dounce homogenizer. The suspension was then centrifuged in a Beckman Ti 70 rotor at 4°C at 35,000 rpm for 60 min. The supernatant from this spin was the starting material for the purification of 'soluble' P-selectin. The pellet was resuspended in 30 ml 1 mM MnCl₂, 1 mM CaCl₂, 5 mM benzamidine-HCl, 0,1M NaCl, 20 mM Tris, 2% Triton X-100, pH 7.5, homogenized 10 strokes with a Dounce homogenizer, and incubated for 1 hr at 4°C. The suspension was then centrifuged for 1 hr in a Beckman Ti 70 rotor at 4°C at 35,000 rpm. The supernatant from this spin was the starting material for the isolation of 'membrane-bound' P-selectin.

Isolation of P-Selectin from Fractionated Platelets: 'Soluble' and 'membrane-bound' P-selectin were then further purified separately by some or all of the following procedures. The non-ionic detergent Rennex 30 (Accurate Chemical and Scientific Corp.) was included at a concentration of 0.1% in all buffers for isolation of the membrane-bound form of P-selectin. All buffers also included 1 mM CaCl₂ and 1 mM MnCl₂. Fractions containing P-selectin were detected by Western blotting with the monoclonal antibody PNB1.6.

Lentil Lectin Chromatography: 'Soluble P-selectin' supernatant or 'membrane-bound P-selectin' detergent extract from 25 units of platelets were passed over a column (1.5 x 5.5 cm) of lectin from Lens culinaris (lentil lectin) coupled to Sepharose™⃝ 4B (Sigma Chemical Co., L-0511). The column was then washed with 150 ml 20 mM Tris, 0.1M NaCl, pH 7.5. Bound glycoproteins were then eluted from the column with 10 x 3 ml aliquots of 20 mM Tris, 0.5M NaCl, 0.5M alpha-methyl D-mannopyranoside, pH 7.5. Fractions containing P-selectin were concentrated to 1.5-2 ml in an Amicon Centriprep™⃝ 30.

Gel Filtration Chromatography: Concentrated fractions from lentil lectin chromatography were injected onto a Toso Haas G3000 SW HPLC column (21.5 mm x 30 cm) equilibrated with 20 mM Tris, 0.1M NaCl, pH 7.5. The column was eluted with the same buffer at a flow rate of 0.75 ml/min, fractions (1.5 ml) were collected, and analyzed by Western blotting. Fractions containing P-selectin were pooled and concentrated to 1.5-2 ml in an Amicon Centriprep™⃝ 30, followed by several buffer exchanges with 20 mM Tris, pH 7.5.

Heparin-Agarose Chromatography: The sample, in 20 mM Tris, pH 7.5, was applied to a 1 ml column of Heparin-Agarose, the column was washed with the same buffer, and eluted with 20 mM Tris, 1.5M NaCl, pH 7.5.

Cation Exchange Chromatography: Pooled fractions from above were exchanged into 20 mM Tris pH 7.5, and injected onto a Toso Haas CM-5PW HPLC column (21.5 mm x 15 cm) equilibrated with 20 mM Tris pH 7.5, and eluted with a salt gradient to 1M NaCl. Fractions containing P-selectin were pooled, and concentrated in an Amicon Centriprep™⃝ 30 to 0.5-1 ml, and stored at -80°C.

### C. ISOLATION AND ACTIVATION OF FRESH HUMAN PLATELETS

All chemicals used throughout the platelet isolation procedure were obtained from Sigma Chemical Company, St. Louis, MO.
1. 42 ml of blood was drawn from a human volunteer blood donor into a syringe containing 7 ml Acid Citrate Dextrose anticoagulant (ACD).

| Preparation of ACD anticoagulant | |
|---|---|
| Dextrose | 2.0g |
| Sodium Citrate | 2.49g |
| citric Acid | 1.25g |

Bring to 100 ml with distilled water
2. The needle was removed and the blood was transferred to two sterile 50 ml tubes.
3. The tubes were centrifuged in an IECC-6000 centrifuge equipped with a 921 head (radius-17.2 cm.) at 800 rpm (approx. 90xg) for 15 min at room temperature with the brake off.
4. The supernatant was removed using a plastic pipette. The supernatant was removed as close as possible to the buffy layer.
5. The supernatant was centrifuged at 1200 rpm (approx. 300xg) for 6 min.
6. The supernatant was removed.
7. The supernatant was centrifuged at 2000 rpm (approx. I200xg) for 10 min. The platelets appeared as a cell button at the bottom of the tube. The supernatant was discarded.
8. The platelets were washed as follows: 2 ml Tyrode-Hepes Buffer pH 6.5, containing Prostaglandin, E₁(PGE₁ at a final concentration of 100nM was added and the platelets were gently resuspended. A further 10 ml of the same buffer was added and the sample was centrifuged at 3000 rpm for 10 min.

| Preparation of Tyrode-Hepes Buffer | |
|---|---|
| NaCl | 8.0g |
| KCl | 0.2g |
| NaH₂PO₄H₂O | 0.057g |
| MgCl₂6H₂O | 0.184g |
| NaHCO₃ | 0.1g |
| Dextrose | 1.0g |
| HEPES | 2.383g |

Bring to 1 liter with distilled water. Adjust pH to 6.5 with 1N NaOH.
9. Step #8 was repeated once more and then the platelets were washed once in the same buffer without PGH₁.
10. The platelets were counted in a Coulter™⃝ Counter and diluted to 2x10⁸/ml.
11. The pH of the platelet suspension was adjusted to 7.2, and the amount of thrombin (Human thrombin-Sigma) required for maximal activation was determined. There is a certain amount of donor variation, but optimal activation is normally obtained in the range of 0.25-0.5 Units/ml. Maximal activation i.e. maximal thrombin-induced aggregation appears to correspond to maximal expression of P-selectin.
12. The required amount of thrombin was added to the platelet preparation and the mixture was allowed to stand at room temperature for 20 min without stirring to prevent aggregation.

### Example 2

### Preparation of Blocking P-selectin Antibody

This example describes preparation of mAB PB1.3, a monoclonal antibody to P-selectin that inhibits binding of thrombin-activated platelets to neutrophils.

One RBF/DnJ male mouse, received from Jackson Laboratories, was used as a source of antibody producing cells and was immunized according to the following schedule (all injections were performed intraperitoneally):
1. Month 1 - 200 µl of packed "outdated platelets" containing approximately 6x10⁹ platelets
2. Month 2 - 250 µl of packed "outdated platelets" (8x10⁹ platelets)
3. Month 4 - 250 µl of packed "outdated platelets" (8x10⁹ platelets)
4. Month 5 - 'Soluble' fraction of P-selectin, isolated on lentil lectin.
5. Month 6 - 'Soluble' P-selectin, purified by lentil lectin, gel filtration, heparin-agarose, and ion-exchange chromatography.
Details of the preparation of all agents used for immunization are presented in Example 1.

### A. MYELOMA FUSION CELL LINE

FOX-NY, a mouse myeloma cell line deficient for adenosine phosphoribosyltransferase (APRT) and hypoxanthine phosphoribosyltransferase (HPRT) was obtained from the American Type Culture Collection (CRL 1732) and maintained in RPMI 1640 containing 10% fetal bovine serum (Hyclone) and 1% L-glutamine.

### B. CELL FUSION PROCEDURE

Four days after the final boost the spleen was removed and 1.2x10⁸ splenocytes were recovered. These were fused with FOX-NY myeloma cells using PEG 1500(BMB) using the following protocol: The isolated splenocytes were washed twice in serum-free cell culture medium. Splenocytes and myeloma cells were combined at a ratio of 1:4.8 (myelomas to spleen cells). The combined cell pellet was washed twice in serum free medium, then aspirated to dryness. The cell pellet was resuspended by gentle tapping and heated in a waterbath at 37°C for 1 min. The pellet was distributed around the sides and bottom of a 50 ml conical centrifuge tube. One ml of PEG 1500 (50% w/v in 75mM HEPES, BMB Lot #14702800) previously warmed to 37°C was added over 60 seconds while rotating the tube to maintain a thin layer of cells. One ml of serum free medium was added slowly over 60 seconds. An additional 1ml of medium was added slightly faster. A further 8 ml of medium was added and the tube was allowed to stand undisturbed for 8 min and was then centrifuged for 5 min at 300xg. The final pellet was resuspended in RPMI 1640 containing 10% fetal bovine serum Hyclone), 1% L-glutamine, 1% Sodium Pyruvate, and 1xAAT (Sigma). The cells were plated in 10 flat bottom 96 well microtiter plates (COSTAR™⃝). No feeder cells were used.

### Example 3

### Preparation of Non-blocking Anti-P-selectin antibody

This example describes preparation of mAB PNB1.6 a monoclonal antibody to P-selectin that does not inhibit binding of thrombin-activated platelets to neutrophils.

One RBF/DnJ male mouse, received from Jackson Laboratories, was used as a source of antibody producing cells and was immunized according to the following schedule (all injections were performed intraperitoneally):
1. Month 1 - 100µl of thrombin-activated freshly isolated platelets (approximately 3x10⁹ platelets)
2. Month 2 - 200µl of thrombin-activated fresh platelets (6x10⁹ platelets)
3. Month 4 - 100µl of thrombin-activated fresh platelets (3x10⁹ platelets)

Four days post final boost, the spleen was removed and the splenocytes were recovered. They were fused with FOX-NY myeloma cells using PEG 1500(Sigma) generally as described in Oi et al., "Immunoglobulin-Producing Hybrid Cell Lines" in Selected Methods in Cellular Immunology, eds. Mishell and Shiigi, pp 351-372, 1980.

### Example 4

### Screening for Antibody

This example describes screening of supernatant media from fused cells produced in Examples 2 and 3. Supernatants from Example 2 were tested by ELISA assay against (a) thrombin-activated platelets, (b) purified P-selectin and (c) recombinant P-selectin. Supernatants from Example 2 were tested by ELISA assay against thrombin-activated platelets. Each of these assays is described below. Preparation of the reagents used in each of the assays is described in Example 1.

### A. THROMBIN-ACTIVATED PLATELETS

1. A 96 well flat bottom COSTAR™⃝ plate was coated with 0.1% gelatin (2% gelatin-Sigma) by adding 100µl/well and incubating at 37°C for 15 min.
2. The gelatin was removed and 100 µl of thrombin activated platelets (10⁸/ml) were added to each well.
3. The plate was incubated at 37°C for 15 min.
4. The plate was centrifuged at 800 rpm (90xg) for 2 min.
5. The unbound platelets were removed and the plate was washed x2 with PBS.
6. 100µl of PBS containing 1% BSA were added to each well and the plate was allowed to stand at room temperature for 60 min.
7. 100µl of supernatant were added to each well. The plate was placed on a shaker at room temperature for 60 min.
8. The plate was washed x4 with PBS.
9. 100µl of peroxidase-conjugated goat anti-mouse IgG diluted 1/1000 in PBS containing 1% BSA was added to each well. The plate was allowed to stand at room temperature for 30 min.
10. The plate was washed x7 with PBS.
11. 100µl of ABTS™⃝ substrate system (Kirkegaard and Perry, Laboratories, Inc.) was added to each well.
12. The plate was allowed to develop at room temperature for up to 30 min.
13. The OD was measured at 414nm in a Titertek™⃝ Multiskan MCC/340.

### B. PURIFIED P-selectin

1. 'Soluble-' or 'membrane-bound' P-selectin, isolated by chromatography on lentil lectin, gel filtration, and DEAE columns, was diluted (1:50 to 1:1000) in DPBS (Dulbecco's Phosphate Buffered Saline is Phosphate-buffered saline (PBS) containing CaCl₂(1mM) and MgSO₄ (0.5mM)) and coated overnight at 4°C onto Falcon™⃝ 96 well microtiter plates.
2. The plates were then blocked for 1 hr or longer with DPBS+1%BSA.
3. Supernatants to be assayed were then added to the wells, and incubated for 30-60 min at room temp.
4. The plates were washed with DPBS, and then sheep anti-mouse IgG horseradish peroxidase conjugate (Sigma A-6782, 1:1000 in DPBS+1%BSA) was added to the wells, and the plates were incubated at room temp. for 1 hr.
5. The plates were then washed with DPBS, and developed with the TMB Microwell Substrate System (Kirkegaard and Perry Laboratories, Inc.)

### C. RECOMBINANT P-selectin

1. Ninety six well flat bottom plates were coated overnight at 4°C with 100µl of P-selectin which had been shed into the serum free medium of a pool of 293 cell clones transfected with the recombinant P-selectin gene. This supernatant contained at least 8ng/ml of P-selectin and was coated onto COSTAR plates either undiluted or diluted 1:8 with media.
2. The plate was washed once with Dulbecco's PBS (DPBS) and blocked with 200µl DPBS containing 1% BSA for 30 minutes at room temperature.
3. The plate was then processed for the ELISA Assay according to steps 7-13 in Section A, Page 9.

### D. ISOTYPING ANALYSIS OF mABs to P-Selectin

Isotype analysis of mAb PB1.3 and PNB1.6 indicated that both monoclonal antibodies were of the mouse immunoglobulin subclass, IgG1. Isotyping was performed using the capture method of Boehringer Mannheim Mouse-Hybridoma-Subtyping Kit,

### Example 5

### Detection of P-selectin

This example describes the detection of P-selectin by Western blotting. Samples containing P-selectin were mixed with an equal volume of SDS-PAGE sample buffer (non-reducing), heated at 100°C, run on a Novex 8-16% gradient gel, and transferred electrophoretically to nitrocellulose. The nitrocellulose membrane was then blocked for at least 1 hr in phosphate-buffered saline (PBS) +1% bovine serum albumin (BSA). The membrane was then incubated at room temperature for 1 hr or longer with either tissue culture supernatant from hybridomas expressing the appropriate monoclonal antibody or purified antibody (5 µg/ml) in PBS+1%BSA. The membrane was then washed several times in PBS+1%BSA, and incubated for 1 hr with a 1 to 1000 dilution of sheep anti-mouse IgG horseradish peroxidase conjugate (Sigma A-6782) in PBS+1%BSA. The membrane was then washed, and bands visualized with tetramethylbenzidine/membrane enhancer (Kirkegaard and Perry Laboratories, Inc.)

For tissue culture supernatants from both PB1.3 and PNB1.6 a protein was detected having a molecular weight of 140Kd. This was shown in preparations of solubilized platelet membranes as well as in purified P-selectin.

### Example 6

### In Vivo Inhibition of Acute Inflammatory Injury by Blocking P-selectin Antibody

This example provides data demonstrating the ability of blocking P-selectin antibody of the invention to treat acute lung injury. In this example, systemic complement activation was produced by vascular infusion of the cobra venom factor (CVF) into rats. Injury in this model develops rapidly and is known to be dependent on toxic oxygen products generated from neutrophils.

For these studies, the two monoclonal antibodies described in Examples 2 and 3 were used. Of particular importance for this study, the blocking P-selectin antibody PB1.3 also inhibited adhesion of thrombin-activated rat platelets to human neutrophils (data not shown). These data suggest that PB1.3 recognizes a conserved functional P-selectin epitope and that a blocking P-selectin antibody might be useful in the treatment of other inflammatory injuries.

For the in vivo experiments 20 units CVF/kg body weight were infused intravenously into 300 gm adult male Long-Evans rats. This results in rapid intrapulmonary, intravascular sequestration of blood neutrophils, with resulting damage of interstitial capillary endothelial cells at points of physical contact of endothelial cells and neutrophils. When employed, PB1.3 and PNB1.6 were infused intravenously in the amounts indicated together with CVF, in total volume of 0.5 ml. Negative control animals received 0.5 ml intravenous infusion of phosphate buffered saline (PBS). In all cases the infusion material also contained trace amounts of [¹²⁵I]-bovine serum albumin and homologous [⁵¹Cr]-red blood cells (RBC). The parameters of lung injury (leakage of albumin and extravasation of RBC) were determined at 30 min. according to established techniques Mulligan, et al., J. Clin Invest. 88:1396 [1991].

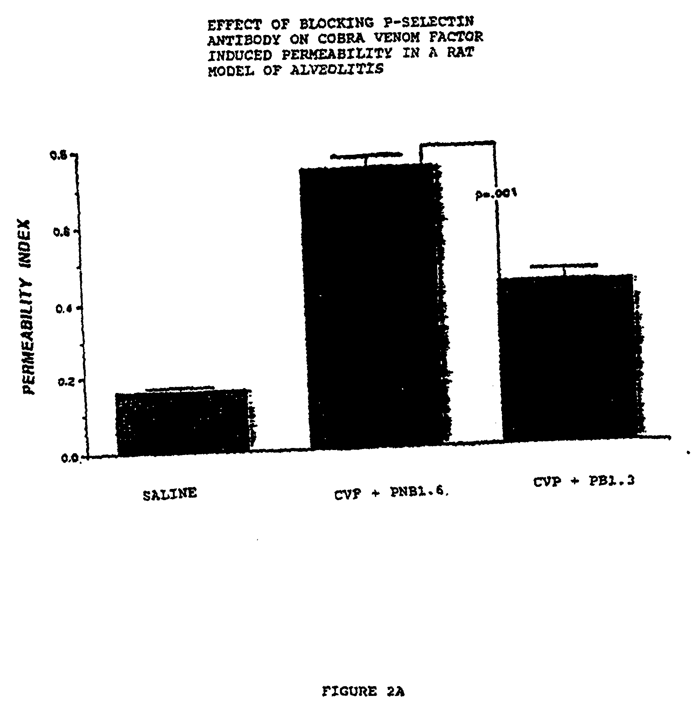

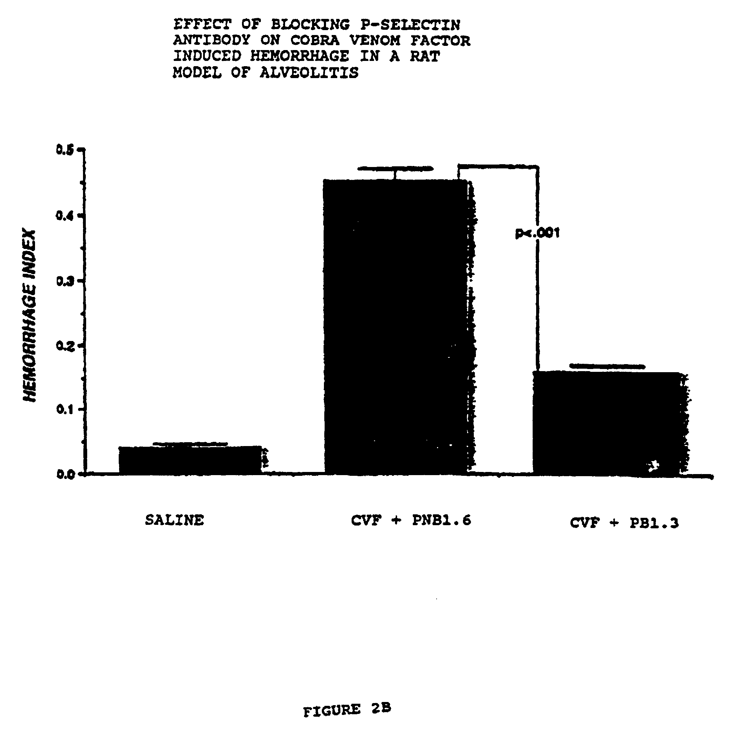

The results of these experiments are shown in Figs. 2A and 2B. Coinfusion of 200 µg PNB1.6 (the non-blocking anti-P-selectin antibody) together with CVF failed to cause any reduction in lung injury when compared to the values of untreated CVF positive controls. Thus animals receiving CVF PNB1.6 in PBS served as the reference positive control values. When 100 µg PB1.3 antibody was infused with the CVF permeability was reduced by 19.2% (p=.002) and hemorrhage was reduced by 37.5% (p=.001) (data now shown). When 200 µg PB1.3 was added, permeability was reduced by 50.8% (p<.001) (Fig. 2A) and hemorrhage by 70.0% (p<.001) and 70.1% (p<.001), respectively (Fig. 2B).

Lungs from companion sets of animals were homogenized sonicated and the myeloperoxidase activity (MPO) measured in order to obtain an estimate of the neutrophil content in lung. MPO was determined by the decomposition of H₂O₂ in the presence of o-dianisidine according to standard procedures, Warren et al. J. Clin. Invest. 84:1873 [1989]. As shown in Fig. 3, treatment with 100, 200 or 400 µg PB1.3 reduced MPO content below the values in the reference (untreated) positive control group by 26% (p=.024), 41% (p<.001) and 50% (p<.001), respectively. In animals injected with 200 µg of PNB1.6 there was no reduction in MPO content (Fig. 3), consistent with the inability of this antibody to protect against CVF-induced lung injury. Thus, the protective effects of the blocking antibody PB1.3, correlate with its ability to interfere with neutrophil accumulation in lung tissue. Transmission electron microscopic examination of lung sections confirmed that treatment with PB1.3 resulted in reduced accumulation of neutrophils within the pulmonary interstitial capillaries, diminished adherence of neutrophils to endothelial cells, and reduced evidence of damaged endothelial cells.

In order to investigate lung expression of P-selectin following intravenous injection of CVF, an additional group of rats was infused with CVF and animals sacrificed at times 0, 5, 10, 15, 20 and 60 minutes later. Lungs were inflated with O.C.T., snap frozen, and sections obtained and examined for presence of P-selectin by immunohistochemical techniques, using PB1.3. Little detectable reactivity in the pulmonary vasculature was found at time 0, whereas staining was clearly evident at 5 minutes and was increased at 15 and 20 minutes after infusion of CVF. The pattern of staining involved pulmonary venules and septal areas in a pattern consistent with staining of interstitial capillaries. At 60 min. staining had largely disappeared (data not shown). Although P-selectin is presumably present in intracellular storage granules prior to CVF infusion, it is apparent that mobilization of P-selectin to the surface of the endothelium dramatically enhanced its reactivity with PB1.3 antibody.

### Example 7

### Competitive Binding Assay

Methods of isolation of platelets from human blood, activation of isolated platelets with thrombin and isolation of PMNs (neutrophils) are described in Example 1 above. INCUBATION OF THROMBIN ACTIVATED PLATELETS WITH MONOCLONAL ANTIBODIES TO P-SELECTIN.
a. Place 20 µl thrombin-activated platelets (2x10⁸/ml) into each of 24 Eppendorf™⃝ tubes (1.5 ml) in duplicate.
b. To 8 of these tubes, add 20 µl of the IgG fraction purified from PNB1.6 at a concentration of 10 µg/ml in Tyrode-Hepes Buffer, pH 7.2 containing 1 mM CaCl₂. To a second row of 8 tubes, add 20 µl of the same IgG preparation at a concentration of 1 µg/ml. To the third row of 8 tubes add 20 µl of the buffer alone.
c. Mix and stand at room temperature for 20 minutes.
d. To each row of 8 tubes add 20 µl of the IgG fraction purified from PB1.3 at concentrations ranging from 10 µg/ml to 0.03 µg/ml.
e. Mix and stand at room temperature for 20 minutes.
f. Add 20 µl neutrophils at 3x10⁶/ml.
g. Mix and stand at room temperature for 20 minutes.
h. Evaluate adhesion microscopically as follows: count 100 neutrophils in each sample. Score a cell as positive if it has bound 2 or more platelets, and as negative if it has bound less than 2. Calculate the percentage of positive cells.

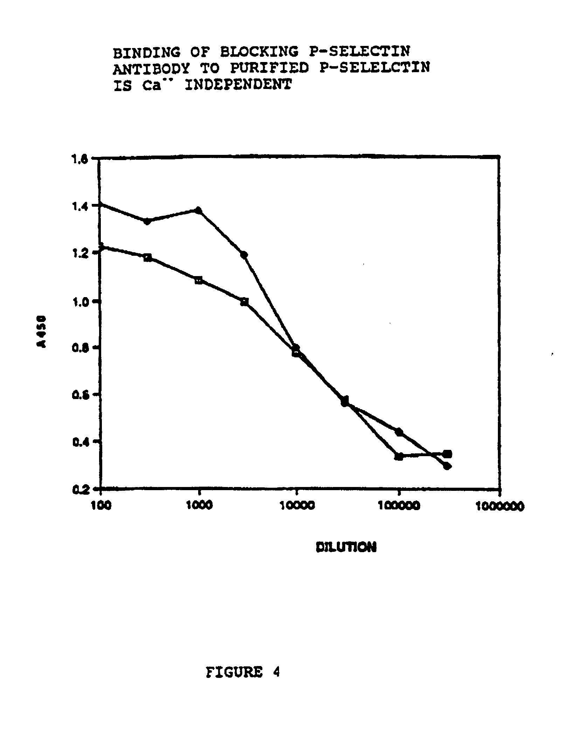

As shown in Fig. 4, pretreatment of thrombin activated platelets with PNB1.6 did not affect the ability of PB1.3 to block their P-Selectin mediated adhesion to neutrophils.

### Example 8

### Characterization of Binding of Blocking P-selectin Antibodies

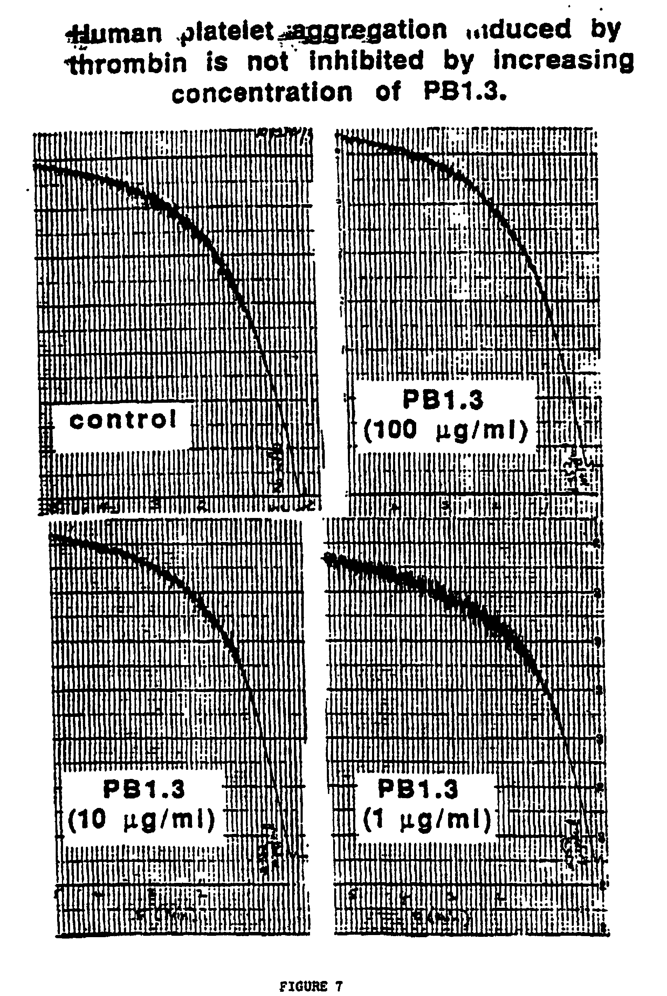

I
   (a) Purification of Monoclonal Antibodies: The monoclonal antibodies PNB1-6 and 84/26 were isolated from tissue culture supernatants by passage over a column of protein G Sepharose™⃝ 4 Fast Flow (Pharmacia). The column was washed extensively with phosphate-buffered saline (PBS), and bound antibody was eluted with 0.1M glycine-HCl pH 2.7 into tubes containing 0.2 to 0.5 volume 1M Tris, pH 8.8. The procedure used for isolating PB1.3 was identical except that the antibody bound to protein G was eluted with 0.1M acetate-HCl pH 2.5 into tubes containing 0.3 volumes 2M Tris, pH 10. Antibody containing fractions were pooled, and dialyzed against several changes of PBS at 4°C.
   (b) Preparation of PB1.3 Affinity Column: For use in the purification of P-selectin, PB1.3 was coupled to tresyl activated agarose according to the manufacturer's affinity instructions (Schleicher and Schuell).
   (c) Purification of P-Selectin: to washed, outdated human platelets, prepared as described above, was added 1/100th volume 5 mg/ml leupeptin, and 1/100th volume 35 mg/ml AEBSF (Calbiochem). After mixing, the platelets were freeze-thawed three times in either a dry-ice/methanol bath or liquid nitrogen, homogenized ten strokes with a Dounce homogenizer, and centrifuged for 1 hr at 35,000 rpm at 4°C in a Beckman 70TI rotor. The pellet was resuspended in Dulbecco's phosphate-buffered saline (DPBS, Whittaker Bioproducts, which contains approximately 0.9 µM Ca²⁺ and 0.5 mM Mg²⁺), and made 5 mM in benzamidine-HCl, 100 µM in leupeptin, and 2% in Triton X-100. The suspension was resuspended, and homogenized 10 strokes with a Dounce homogenizer. After a 1 hr incubation at 4°C, the solution was again centrifuged in a Beckman 70TI rotor for 1 hr at 4°C at 35,000 rpm. The supernatant was passed over a column of PB1.3-agarose that had been equilibrated with DPBS+0.05% Rennex 30 (Accurate Chemical and Scientific Corp.). The column was washed extensively with DPBS+0.05% Rennex 30, and bound P-selectin was eluted with 0.1M triethylamine-HCL, 0.05% Rennex 30, pH 11.5, into tubes containing 0.1 volume 1 M phosphate, pH 6.8. Fractions containing P-selectin were pooled, dialyzed against DPBS+0.05% Rennex 30, concentrated using either a Centricon 30™⃝ or Centriprep 30™⃝ spin concentrator (Amicon™⃝), aliquoted, and stored at -80°C.
   (d) Preparation of the Peptide CQNRYTDLVAIQMKNE (Cys-Gln-Asn-Arg-Tyr-Thr-Asp-Leu-Val-Ala-Ile-Gln-Asn-Lys-Asn-Glu-NH₂₎. The peptide was synthesized on an Applied Biosystems (Foster City, CA) 430A peptide synthesizer using Fmoc protected amino acids and 2-(1H-Benzotriazol-1-yl)-1,1,3,3-tetramethyluronium hexafluorophosphate (HBATU) esters for amino acid activation. Each amino acid was routinely double coupled. Fmoc protected amino acids and Hydorxybenzotriazole were purchased from Applied Biosystems. All solvents were purchased from Burdick and Jackson. HBTU was purchased from Richelieu Biotechnologies (St-Hyacinthe, Canada). Piperidine and trifluoroacetic acid, acetic anhydride, thioanisole, phenol and ethanedithiol were purchased from Sigma Chemical Corporation.
      Fmoc-Amide resin (Bachem Biosciences) was loaded into the peptide synthesis reaction vessel and washed one time with N-methylpyrolidone (NMP). The following operations were then sequentially performed:
      1. The Fmoc protecting group was removed by treatment of the resin bound amino acid with 25% piperidine in NMP.
      2. The resin was washed 5 times with NMP.
      3. A mixture containing N-α-Fmoc-L-Glutamic acid γ-t-butylester, diisopropylethylamine, HBTU and NMP was added to the reaction vessel and allowed to react for 30 minutes, under vortex agitation.
      4. The solvent was drained, and the resin was washed three times with NMP.
      5. Steps (3) and (4) were repeated two more times.
      6. The resin was washed four more times with NMP. Steps 1-6 were repeated for each amino acid of the peptide. Following the final coupling cycle, the resin-bound peptide was deprotected by reaction with 25% piperidine in NMP, washed 7 times with NMP, and washed 2 times with dichloromethane. The resin was dried in vacuo for 24 hours. The peptide was cleaved from the resin by treatment with trifluoroacetic acid containing 2.5% ethanedithiol, 5% thioanisole, 7.5% phenol, and 5% water. The polystyrene resin was separated from the trifluoroacetic acid solution by filtration. Trifluoroacetic acid was removed by evaporation in vacuo. The crude peptide was triturated with diethylether and dissolved in water. The water was removed by lyophilization. The peptide was then purified by reverse phase HPLC on a C₈ column (VYDAC) using a gradient of acetonitrile, water, each containing 0.1% TFA as modifier.
   (e) Biotinylation of Monoclonal Antibodies: Antibodies were dialyzed against 0.1M sodium bicarbonate, pH 8.5. NHS-LC Biotin (Pierce) was added to 0.3 mg/ml. After 2 hrs at room temperature, antibodies were dialyzed against several changes of PBS.
   (f) Coating of Microtiter Plates for ELISA: 96 well microtiter plates (Falcon Microtest III) were coated overnight at 4°C with 50 µl/well of 2 µg/ml affinity purified P-selectin in DPBS.
   (g) Western Blotting: PB1.3, PNB1.6, and 84-26 all bind to a single band of 140 KD when platelets are dissolved in SDS-PAGE running buffer (unreduced), subjected to SDS-PAGE, and transferred to nitrocellulose (data not shown). We have also found that PB1.3 and PNB1.6 no longer recognize P-selectin on western blots when the samples have been reduced with β-mercaptoethanol.
II. Effect of Chelating Divalent Cations on Binding of Monoclonal Antibodies to P-Selectin: Two microtiter plates were coated with affinity purified P-selectin as above. Plate 1 was blocked with 200 µl/well PBS+1% BSA, while plate 2 was blocked with 200 µl/well DPBS+1% BSA (DPBS contains Ca²⁺ and Mg²⁺). After 1 hr, the plates were washed (all washes for plate 1 were PBS, for plate 2, with DPBS. 25 µl of 25 mM EDTA in PBS+1% BSA was added to the wells of plate 1, and 25 µl of DPBS+1% BSA was added to plate 2. After 1 hr, 25 µl of dilutions of the appropriate antibody were added to the wells of either plate. Dilutions for plate 1 were made in PBS+1% BSA, for plate 2 in DPBS+1% BSA. After 1 hr, the plates were washed, and 50 µl of a 1 to 1000 dilution of sheep anti-mouse IgG horseradish peroxidase conjugate (dilutions for plate 1 were made in PBS+1% BSA, for plate 2 in DPBS+1% BSA) was added. After 1 hr, the plates were washed, and 50 µl of the peroxidase substrate TMB (3,3',5,5'-tetramethylbenzidine, Kirkegaard and Perry Laboratories, Inc.) was added. After the color had developed to the appropriate level, the substrate reaction was quenched by the addition of 25 µl of 1M phosphoric acid and the absorbance at 450 nm was read.
   Fig. 4 shows the binding of the anti-P-selectin monoclonal antibodies to P-selectin in the buffer DPBS, which contains CA²⁺ and Mg²⁺, and in PBS + 25 mM EDTA, a chelator of divalent metal cations. The concentration of divalent cations present in DPBS is more than sufficient to support neutrophil adhesion to P-selectin (Geng et al., 1991), while the chelator EDTA, at 25 mM, is more than sufficient to block neutrophil adhesion to P-selectin. The binding of all of the monoclonal antibodies to P-selectin is little affected by the presence of EDTA, demonstrating the Ca²⁺ is not required to be present for binding to occur. PB1.3 is a blocking antibody, that is, it is able to block the binding of neutrophils to P-selectin, for example on activated platelets. The PNB1.6 is a non-blocking antibody. The ability of 84/26 to block the binding of neutrophils to P-selectin has not yet been fully characterized. In contrast, of the anti-P-selectin monoclonal antibodies described by Geng et al., only the non-blocking antibody S12 is able to bind in the absence of Ca²⁺.
III. Effect of the peptide CQNRYTDLVAIQNKNE on the binding of PB1.3 to P-Selectin: A microtiter plate was coated with affinity purified P-selectin as above, blocked with 200 µl/well DPBS+1% BSA for 1 hr, and washed. Dilutions of PB1.3 in DPBS+1% BSA were mixed with either 0.35 mg/ml CQNRYTDLVAIQNKNE in PBS, or as a control, PBS alone. After 1 hr, 50 µl of each antibody dilution was added to the microtiter plate, and incubated for 1 hr. The plate was washed with DPBS, and a 1 to 1000 dilution of sheep anti-mouse IgG horseradish peroxidase conjugate in DPBS+1% BSA was added. After 1 hr, the plate was washed, the substrate TMB was added, and the color development was stopped and the plates read as above.

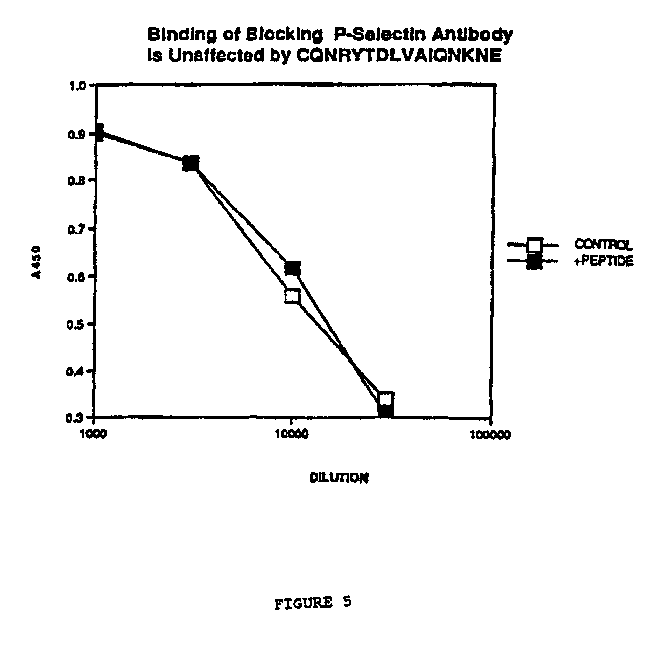

   Fig. 5 shows that the peptide CQNRYTDLVAIQNKNE, homologous to residues 19-34 of the lectin domain of P-selectin, has no effect on the binding of PB1.3 to P-selectin when the peptide is present at a concentration of 0.35 mg/ml. This distinguishes this particular monoclonal antibody from the monoclonal antibodies G1, G2, and G3, whose binding to P-selectin is partially or completely blocked by this peptide.
IV. Ability of Cytel Monoclonal Antibodies PB1.3, PNB1.6, and 84/26 to Block Binding of Each other to P-Selectin: Microtiter plates coated with affinity purified P-selectin as above were blocked for 1 hr with DPBS+1% BSA, and washed. 25 µl of a 50 µg/ml solution of PB1.3, PNB1.6, or 84/26 in DPBS+1% BSA were added to appropriate wells, and incubated for 1 hr. Then, 25 µl of a dilution (in DPBS+1% BSA) of biotinylated antibody was added. The plate was then incubated on a platform shaker for 1 hr. After washing, 50 µl of a 1 to 1000 dilution of a streptavidin horseradish peroxidase conjugate was added, and incubated for 1 hr. After washing with DPBS, the plate was developed with the substrate TMB as above.

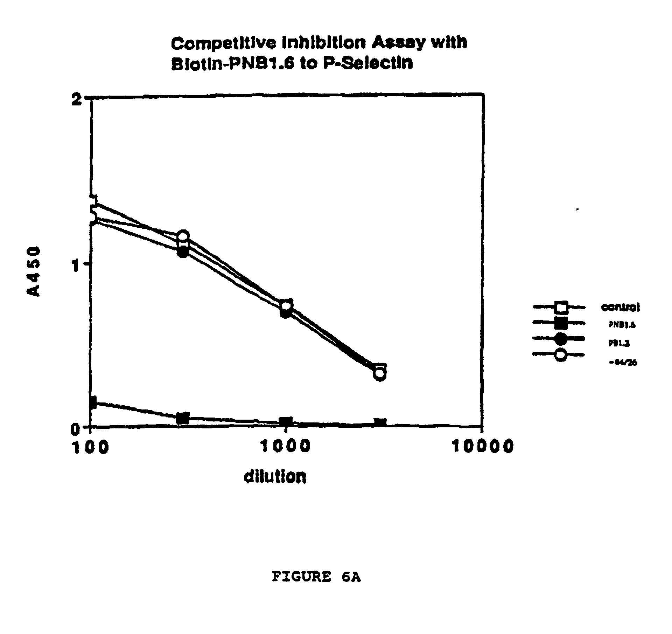

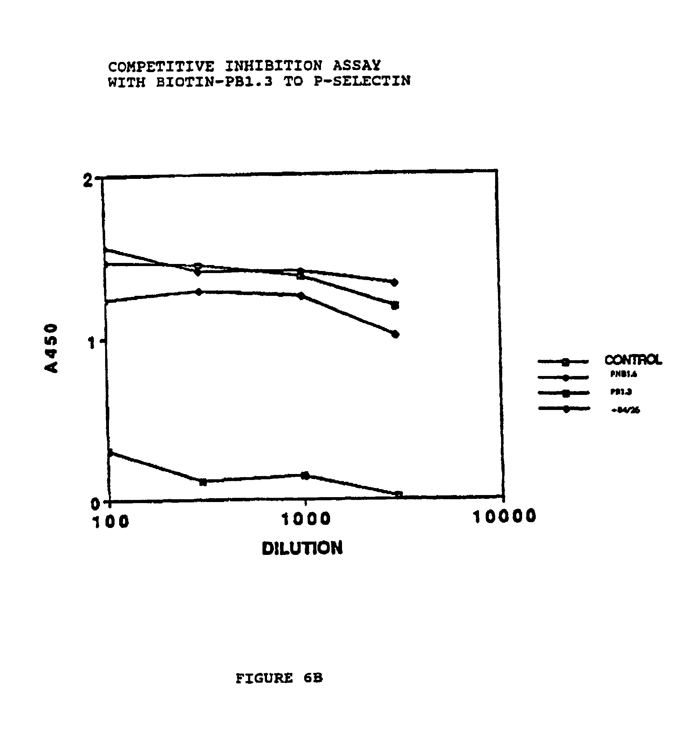

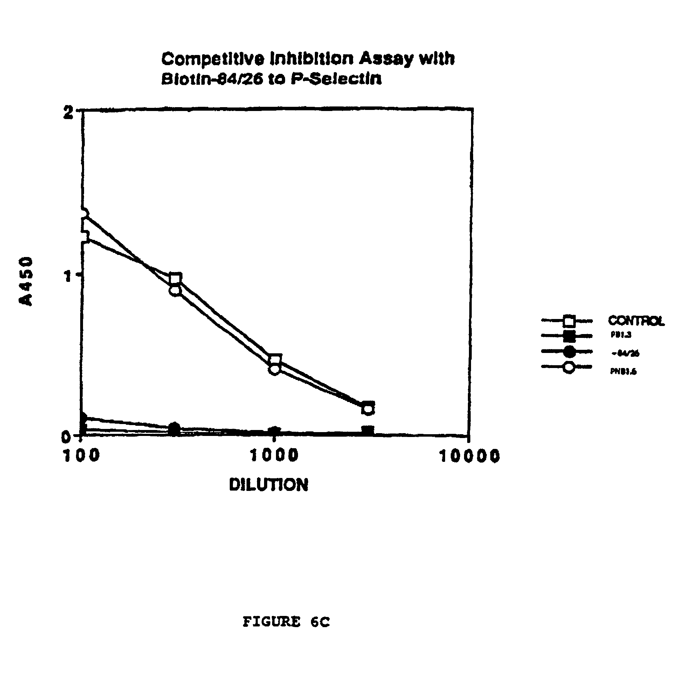

   Fig. 6 shows the ability of the monoclonal antibodies PB1.3, PNU1.6, and 84/26 to interfere with the binding of each other to P-selectin. Fig. 6A shows that only PNB1.6 is able to block the binding of biotinylated PNB1.6 to P-selectin, demonstrating that PB1.3 and 84/26 must recognize different epitopes from PNB1.6. Likewise, In Fig. 6B, PB1.3, but not PNB1.6 or 84/26, is able to block the binding of biotinylated PB1.3 to selectin, demonstrating that the other two monoclonals must recognize different epitopes. Fig. 6C, both 84/26 and PB1.3, but not PNB1.6, are able to block the binding of biotinylated 84/26 to P-selectin.
V. Blocking P-selectin antibodies do not inhibit thrombin-induced aggregation of human platelets.
   This example demonstrates that the P-selectin antibodies, PB1.3 and PNB1.6 do not inhibit thrombin-induced aggregation of platelets. Fresh human platelets were isolated as previously described and suspended in Tyrode-Hepes Buffer, pH 7.2 at a concentration of 2x10⁸/ml.
   Platelet aggregometry was performed in a Lumi aggregometer (Chrono-Log Corp.). For the assay, 0.45 ml of the platelet suspension was placed in a standard siliconized cuvette. To this was added 10 µl of the antibody or vehicle control. After 5 min. at 37°C, 0.1 unit of thrombin (Human, Sigma) was added and aggregation was recorded.
   When platelet aggregation was measured under control conditions, in the absence of antibody, an aggregation response of 71 units was obtained. An equivalent extent of aggregation was obtained in the presence of 10 µl of undiluted culture supernatants from cells producing PB1.3 antibody (Fig. 7). Different concentrations of PB1.3 (1-100 µg/ml) were added to the platelet suspension. At no concentration of PB1.3 was platelet aggregation different from the response determined in the absence of antibody (Fig. 7).

### Example 9

### In vivo Protective Effects of Blocking P-Selectin Antibody, PB1.3 in Feline Myocardial Ischemia and Reperfusion

A model of myocardial ischemia and reperfusion injury in cats was performed according to the methods of Tsao et al. (Circulation 82:1402-1412 [1990]). Briefly, male cats (2.5-3.5 Kg) were anesthetized with sodium pentobarbital (30 mg/Kg, i.v.). An intratracheal tube was inserted through a mid-line incision and all cats were given intermittent positive pressure ventilation by a Harvard small animal respirator. A polyethylene catheter was inserted into the external jugular vein and the right femoral vein was cannulated and connected to a Statham P23Ac pressure transducer for the measurement of arterial blood pressure. A midline thoracotomy was performed, the pericardium was opened and the heart was exposed. A 2-0 silk suture was carefully placed around the left anterior descending artery (LAD) 10-12 mm from its origin. After a 30 min period of stabilization myocardial ischemia was initiated by complete ligation of the LAD for 1.5 hours of ischemia followed by 4.5 hours of reperfusion. Blocking (PB1.3) and non-blocking (PNB1.6) anti-P-selectin antibodies were administered intravenously at a dose of 1 mg/Kg 10 min prior to the initiation of reperfusion.

The ischemic myocardium was determined as that portion of tissue which did not stain with nitroblue tetrazolium and was expressed as a percentage of the area at risk. Area at risk was determined by reocclusion of the LAD at the end of the reperfusion period followed by the injection of Evans blue dye into the left atrium. The area of risk was, therefore, determined by negative staining.

Endothelial dependent relaxation of coronary artery rings was determined by measuring the acetylcholine-induced relaxation of rings previously contracted with the thromboxane A2 mimetic U46619. Data are expressed as percentage relaxation.

Neutrophil accumulation in the myocardium was measured as the myeloperoxidase activity of polytron homogenized myocardial tissue.

The results of these experiments are shown in Fig. 8. In cats treated with the non-blocking anti-P-selectin antibody PNB1.6 the extent of myocardial ischemia was 33 ± 5% of the area at risk. In contrast, the extent of ischemia in PB1.3 treated animals was significantly (P<0.01) less at 15 ± 3% of the area at risk. Endothelial dependent relaxation to acetylcholine was also significantly preserved in ischemic-reperfused coronary arteries taken from cats treated with PB1.3 compared to PNB1.6 (67 ± 6 vs 11 ± 3%, P<0.01). That these phenomena are attributable to a reduction in leukocyte accumulation is suggested by a significant (P<0.1) reduction in the number of PMNs binding to coronary artery endothelium from PB13 treated animals (64 ± 7 PMN/mm²) compared with PNB1.6 treated animals (136 ± 12 PMN/mm²).

### Example 10

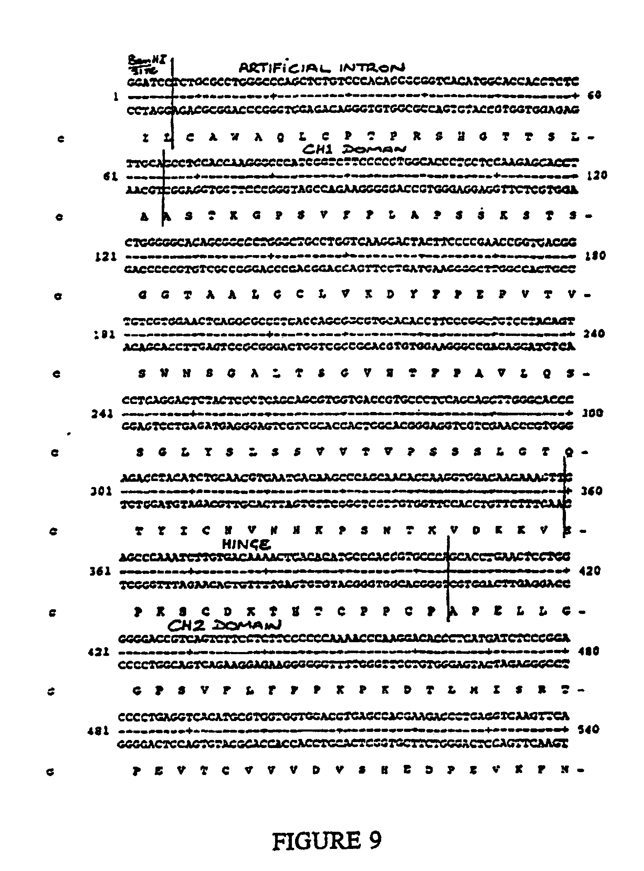

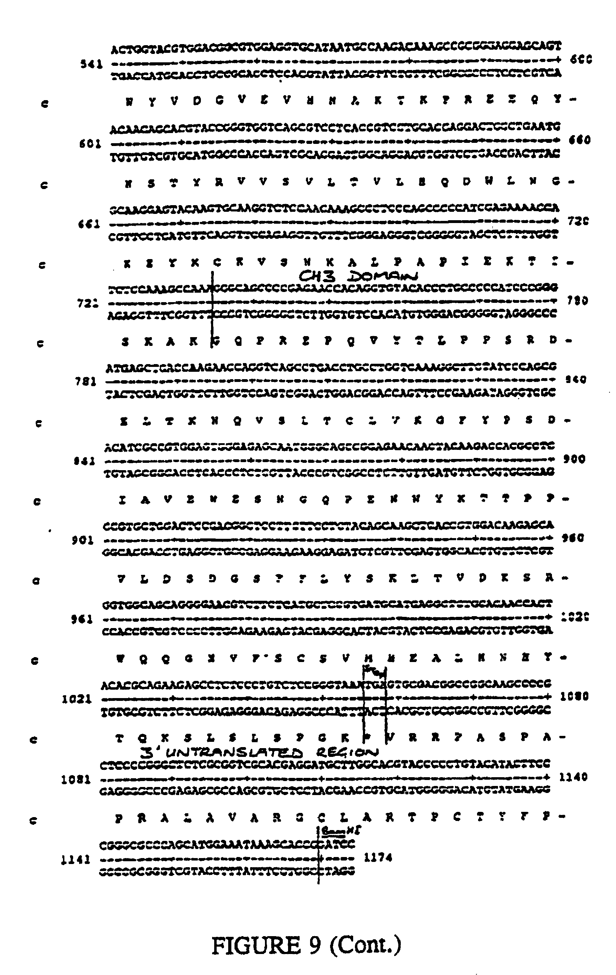

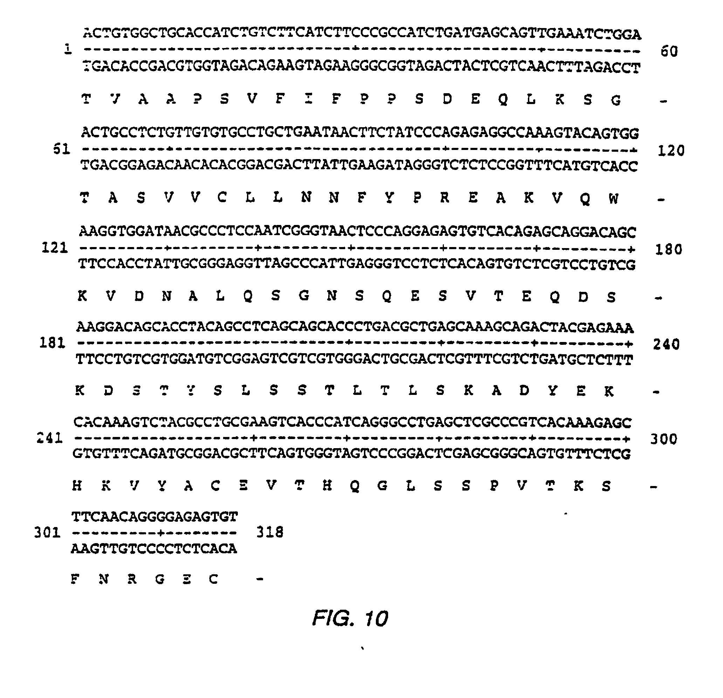

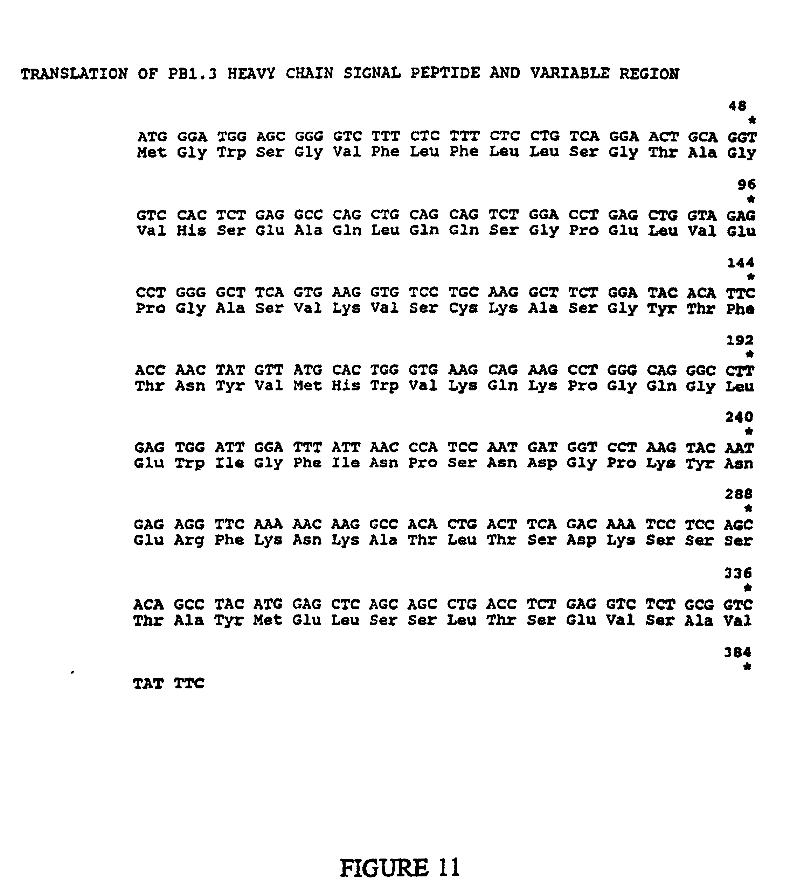

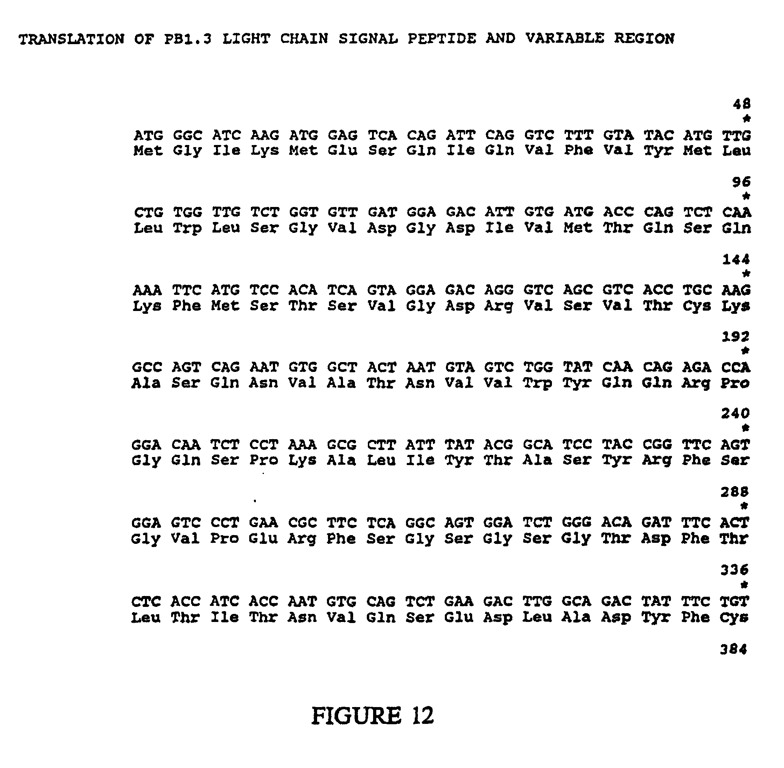

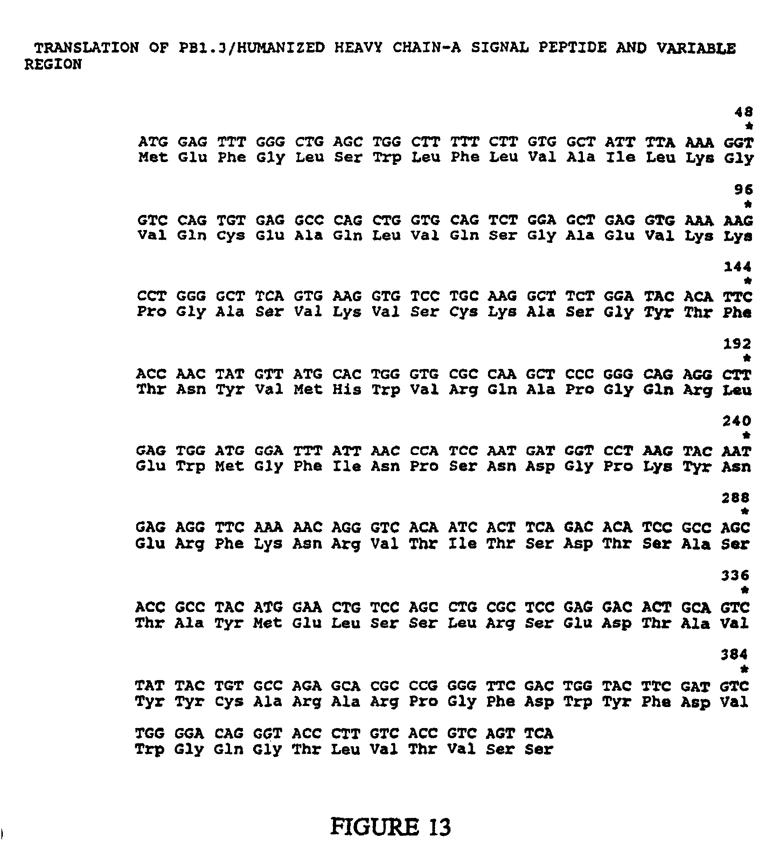

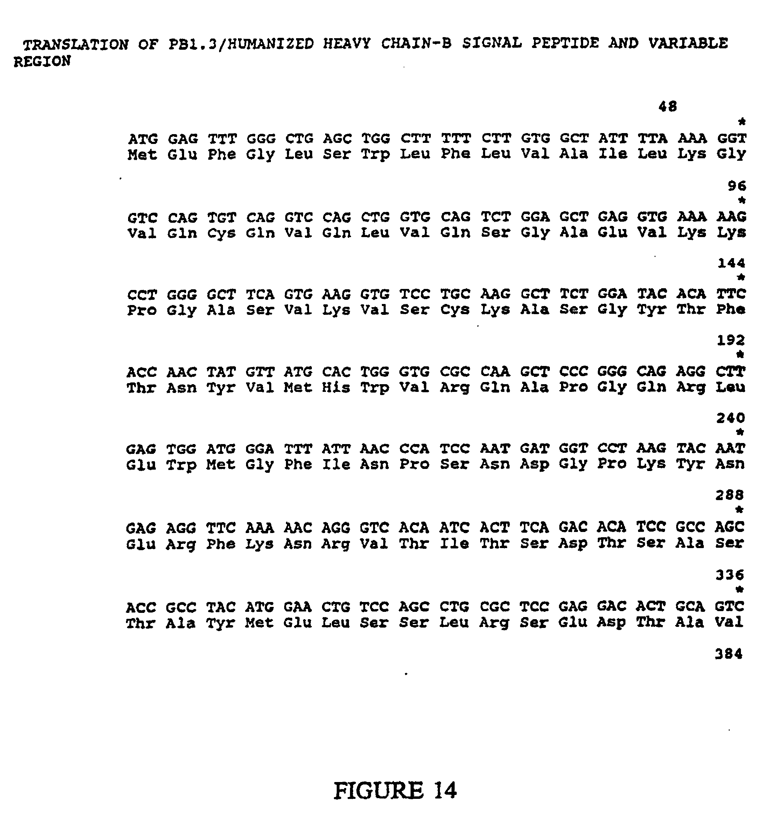

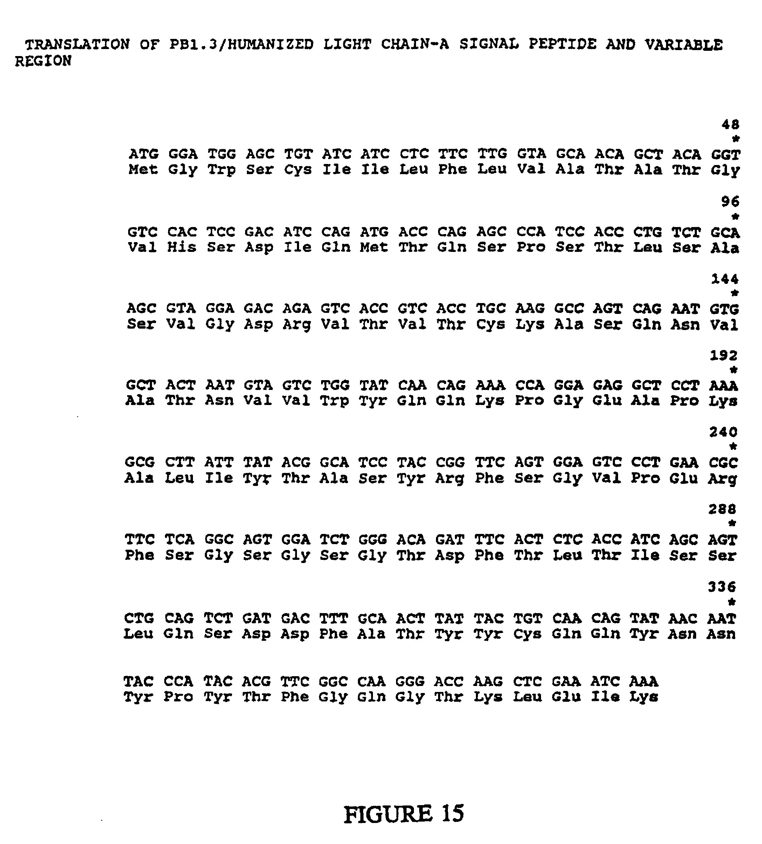

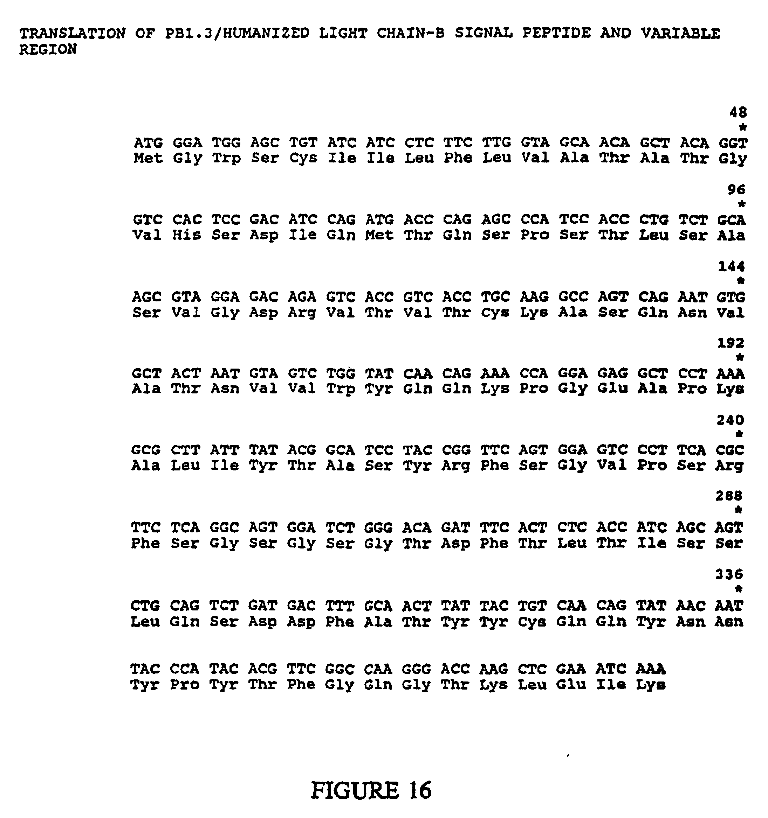

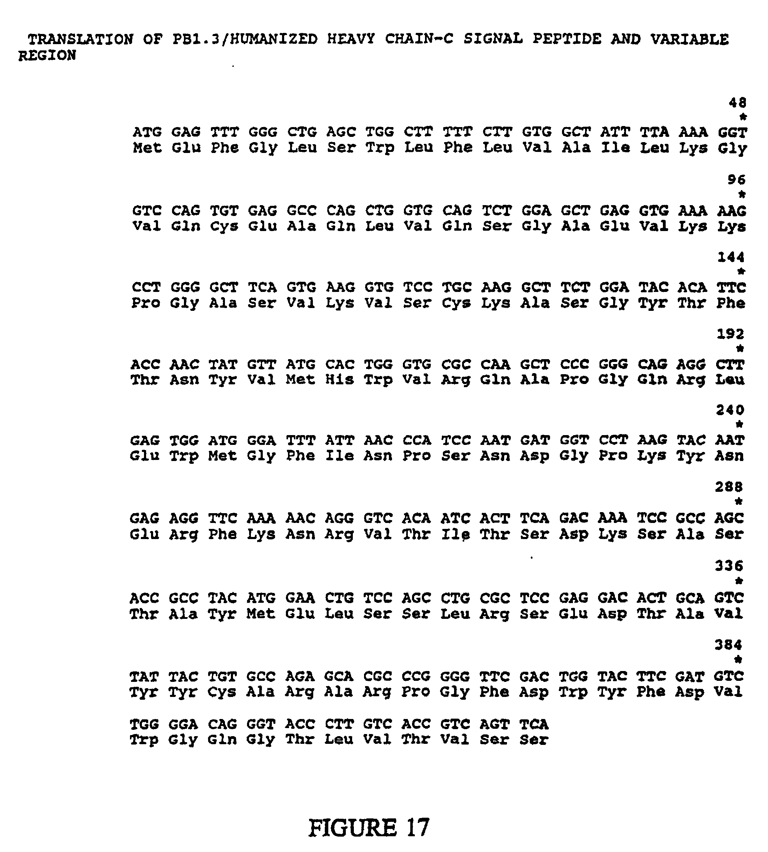

cDNAs encoding the variable regions of the heavy and light chains of murine monoclonal antibody PB1.3 were cloned and characterized by sequencing. The sequences of the variable regions and signal peptides of the heavy and light chains of PB1.3 are given in Figs. 11 and 12. The CDRs from PB1.3 heavy and light chains were substituted for the CDRs of human heavy chain and light chain. The human framework regions were modified using the methodology described in Monoclonal Antibodies, 2: Applications in Clinical Oncology (Editor A. Epenetos; Chapman & Hall, 1992, see especially, Bendig et al. The Humanization of Mouse Monoclonal Antibodies by CDR-Grafting: Examples with AntiViral and Anti-Tumor Cell Antibodies contained therein to generate different versions of the variable regions of the humanized PB1.3. The humanized variable regions were inserted into expression vectors. The sequences of the human constant regions are given in Figs. 9 and 10. The sequences of variable regions and signal peptides of the heavy and light chains of different versions of PB1.3/Humanized are given in Figs. 13, 14, 15, 16 and 17. By coexpressing appropriate combinations of heavy and light chains, several humanized antibodies can be expressed. Coexpression of pHCMV-1748RH_{A-γ}1Ci-dhfr and pHCMV-1748RL_{A}-KR-neo gives rise to the PB1.3/Humanized version A. Coexpression of pHCMV-1747CH-γ1Ci-neo and pHCMV-1747CL-KR-neo gives rise to the PB1.3 chimera.

### Example 11

### EXPRESSION OF PB1.3 AND PB1.3/Humanized IN COS CELLS.

### I Electroporation of COS cells

The DNA constructions were tested in COS cells for transient expression of the PB1.3/Humanized/Chimeric antibody and PB1.3/Humanized antibody. The DNAs were introduced into the COS cells by electroporation using the Gene Pulser™⃝ apparatus (Biorad). COS cells were trypsinized and washed once in phosphate buffered saline (PBS). DNA (10 µg of each heavy chain plasmid and appropriate light chain plasmid) and a 0.8 ml aliquot of 1 x 10⁷ cells/ml in PBS were placed in a sterile Gene Pulser™⃝ Cuvette (Biordad, 0.4 cm gap). A pulse was delivered at 1,900 volts, 25 microfarads capacitance. After a 10 minute recovery period at ambient temperature, the electroporated cells were added to 10 ml of DMEM media (GIBCO) containing 10% fetal calf serum. After a 48 hour incubation, the medium was collected, centrifuged to remove cellular debris, and stored under sterile conditions at 4° C for short periods of times.

### II Enzyme-linked immunoadsorbent assay (ELISA)

Media from the transfected COS cells were assayed by ELISA first to determine the amount of human antibody that was produced and second to determine the amount of antibody bound specifically to P-selectin. Falcon™⃝96 well microtiter plates (Falcon™⃝ 3912, Microtest III) were coated with 50 µl of a solution of recombinant P-stelectin (1.2 mg/ml) or a solution of goat anti-human IgG (whole molecule, Sigma, mg/ml) in Dulbecco's phosphate buffered saline (DPBS). Plates were coated either overnight at 4° C or for 2 hours at 37° C. The plates were then blocked with 200 microliters/well of DPBS + 1% bovine serum albumin (BSA) for one hour or longer. The plates were then washed with DPBS.

Serial dilutions of the monoclonal antibodies in DPBS + 1% BSA were added to the plates (50 microliters/well), and the plates were incubated for one hour at room temperature. The plates were then washed three times with DPBS.

50 microliters/well of a second antibody (goat anti-human IgG peroxidase conjugate, Sigma A-8867) diluted 1 to 1000 in DPBS + 1% BSA was added to the plates, and the plates were incubated for one hour at room temperature. The plates were washed three times with DPBS.

50 microliters of tetramethylbenzidine peroxidase substrate (TMB, from Kirkegaard and Perry Laboratories) was added to each well, and the color was allowed to develop for an appropriate period of time (usually 3 to 15 minutes), and the reaction was quenched by the addition of 50 microliters/well 1M phosphoric acid. The plates were read at 450 nm.

The concentration of the antibodies produced in all COS cell supernatants was determined by comparing the IgG ELISA results to a human polyclonal antibody of known concentration as the standard (Sigma).

### III Data Analysis

P-selectin binding was plotted as optical density at 450 nm against concentration of IgG.

### IV Purification of PB1.3

PB1.3 was purified as described in Example 8.

### Example 12

### The effect of PB1.3 on tissue injury produced by ischemia reperfusion following replantation of the rabbit ear.

New Zealand White rabbits were anesthetized with a combination of ketamine and xylazine followed by infiltration of lidocaine into the base of the ear. The left ear was partially amputated leaving the central artery, vein and a bridge of cartilage intact. All nerves were divided to render the ear totally anesthetic. The ear was then reattached and a microvascular clip was placed across the artery to produce total ischemia. Rabbits were kept in a room maintained at 23.5°C for 6 hours then the microvascular clamp was removed and the ear allowed to reperfuse. Treatment was administered immediately prior to reperfusion with either PB1.3 (2 mg/Kg) or with either saline or an isotype (IgG1) matched murine monoclonal antibody designated PNB1.6 (2 mg/Kg). Ear volume was measured daily for 7 days by water displacement to quantify tissue edema. On day 7 necrosis was estimated as a percentage of total surface area.

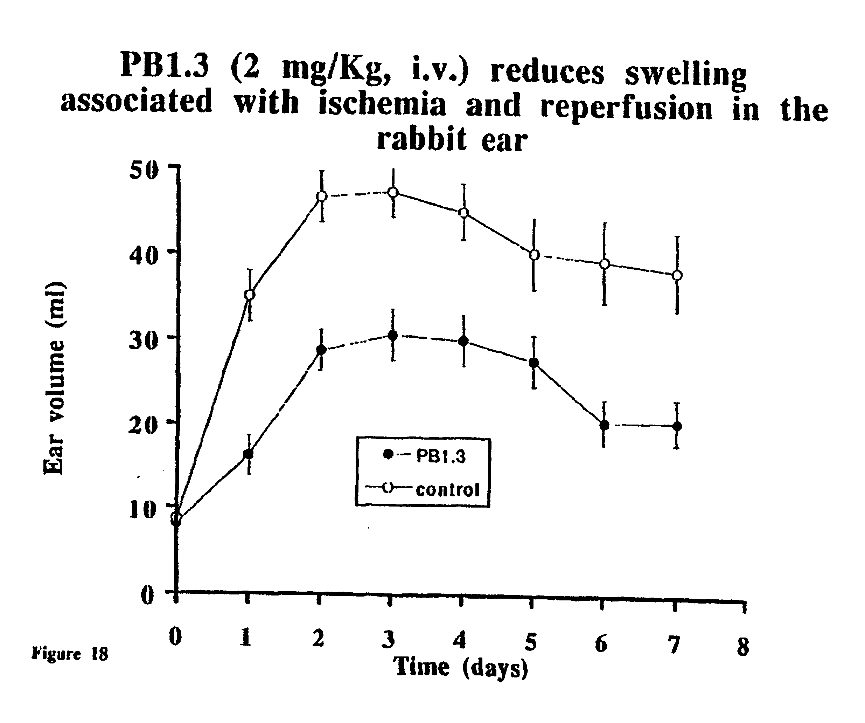

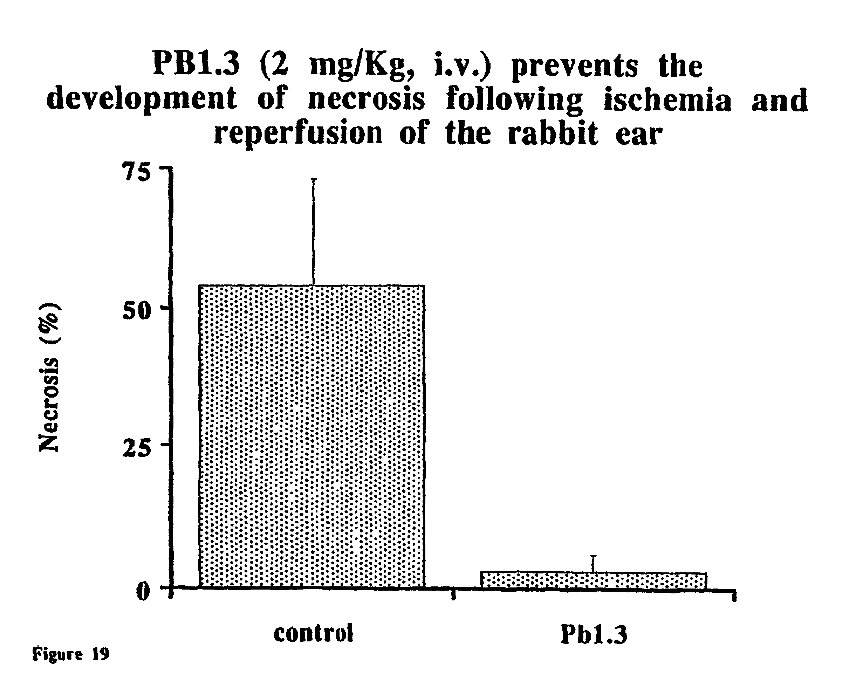

A significant increase in ear volume was determined in control animals administered either saline or PNB1.6 following reperfusion of the ears (Fig. 18). Because no differences between the two control groups were noted, their data have been combined. An increase in edema was also seen in animals treated with PB1.3 the magnitude of the increase was significantly less than that observed in the control group at all time points measured. The extent of necrosis (Fig. 19), measured on day 7, was also significantly reduced in PB1.3 treated animals compared with control animals.

Thus, ischemia followed by reperfusion of the replanted rabbit ear produces an edematous response initially followed by extensive tissue necrosis. These two manifestations of tissue injury are substantially and significantly reduced in animals pre-treated with the anti-P-selectin antibody PB1.3.

### Example 13

### The effect of PB1.3 on vascular patency following ischemia and reperfusion of the canine skeletal muscle.

This experiment utilizes a model of ischemia and reperfusion of the isolated canine gracilis muscle. One aspect of the reperfusion injury is a phenomenon referred to as the 'no-reflow' phenomenon in which blood flow through the microcirculation of a previously ischemic tissue is absent or severely reduced following reperfusion.

An isolated canine gracilis muscle preparation was utilized as described previously (Carden, Smith & Korthuis [1990] Circ. Res. 66 1436-1444). Briefly, adult mongrel dogs were anesthetized with pentobarbital sodium. The skin overlying the gracilis muscle was divided and the muscle was freed from connective tissue by blunt dissection. The obturator nerve was sectioned. The proximal caudal artery and vein were cannulated and all other collateral vessels ligated. The femoral artery cannula was connected to a constant flow perfusion pump and the outflow from the femoral vein drained in to a reperfusion reservoir. Arterial and venous pressures were monitored via side branches and blood flow was adjusted to maintain perfusion pressure at 100 mm Hg. Vascular isolation was assumed complete if arterial perfusion pressure decreased to less than 20 mm Hg when the perfusion pump was switched off.

Microvascular patency was assessed by perfusion of the isolated gracilis muscle with contrast media (india ink) at the end of the experimental protocol. computerized video imaging was used to quantitate the number of ink-containing microvessels (<10 µm diameter) per muscle fiber in histologic sections obtained from isolated canine gracilis muscles subjected to 4.5 hours of continuous perfusion, 4 hours of ischemia followed by 0.5 hours of reperfusion or ischemia reperfusion in the presence of the anti-P-selectin antibody PB1.3 at a concentration of 40 µg/ml in the perfusate.

The ischemia and reperfusion protocol reduced the number of patent microvessels to 36 ± 3 % of the value determined in control animals. When PB1.3 was included in the perfusate the number of patent microvessels following ischemia and reperfusion was 119 ± 18 % of control.

Thus, the anti-P-selectin antibody, PB1.3, completely prevented the development of the 'no-reflow' phenomenon, determined by the number of patent microvessels, in the ischemic and reperfused canine gracilis muscle.

### Example 14

### The effect of PB1.3 on leukocyte endothelial cell interactions induced by the degranulation of tissue mast cells

### I. Methods

### a) Intravital microscopy

Male Wistar rats (200-250g) were obtained from Harlan Sprague Dawley, Indianapolis, and were fasted for 12-24 hrs before the surgical procedure. Surgical anaesthesia was induced by an intramuscular injection of ketamine/rompun/acepromazine. A catheter was placed in the left jugular vein, and a tracheostomy was performed to facilitate spontaneous breathing. The abdomen was shaved and washed, and a 3/4 inch long incision was made through the midline into the peritoneal cavity, taking care to avoid any bleeding. The rat was placed on a microscope stage specifically designed for the intravital microscopy procedure. A well-vascularized posterior loop of the ileum was exteriorized, and the mesentery draped over a viewing pedestal heated to 37°C. Throughout the experiment, the mesentery was superfused at 2 ml/min with a heated bicarbonate-buffered saline solution (37°C, pH 7.4). Additionally, small pieces of Saran-Wrap (previously soaked in alcohol and rinsed in saline) were placed over the ileum and mesenteric areas peripheral to the viewed area, in order to keep these areas moist. The microcirculatory beds were observed through an intravital microscope (Mikron Instruments, San Diego) equipped with 10x oculars and a 20x objective (water immersion, numerical aperture 0.4). A high-resolution video system, consisting of a video camera (Hitachi color CCD) clamped onto the microscope, a video timer (American Video Equipment), a VCR (Sony SVO-9500MD) and a monitor (Sony Trinitron), permitted video recordings of the microscope image. In each mesentery preparation, 3 postcapillary venular segments of approximately 180 µm length were selected for repeated recordings according to the following criteria: 1) A true postcapillary venule receiving blood from confluent capillaries, diameter 15-30 µm; 2) Brisk blood flow, so that individual red blood cells could not be distinguished; 3) Some baseline rolling of leukocytes (less than 8 leukocytes present at any given time in the vascular segment), but no firmly adhering leukocytes. A leukocyte was defined as being firmly adhering if it stayed immobile at the vessel wall for more than 30 seconds.

### b) Experimental protocol

One-minute video recordings of the baseline leukocyte interaction in the selected venules were made at 20 and 25 min after completion of the surgical procedure. All treatments were administered intravenously before the second baseline recording. At 30 min after surgery, infusion of compound 48/80 (Sigma, catalog # C4257) into the superfusion buffer was begun, and continued throughout the rest of the experiment. This yielded a final concentration of 10 µg/ml at the mesentery, and was done in order to induce mast cell degranulation. Venules were recorded again at 20 and 30 min after the beginning of 48/80 application. The extent of leukocyte-endothelial cell interactions was determined at the end of the experiment by analyzing the recorded video tapes. The number of clearly visible leukocytes (i.e., cells interacting with the vessel wall) was determined from frames frozen at exactly 0, 15, 30, 45, and 60 seconds of the recorded sequence, and the mean from these five determinations was calculated. Hence, counted leukocytes included rolling cells as well as those firmly adherent.

### c) Statistics

Each experimental group contained 5-6 animals with 2-3 venules from each animal. The mean and its standard error is given. Significant differences among group means were tested through analysis of variance followed by the Tukey-Kramer's HSD test. Differences before and after treatments in individual venules were studied by paired t-tests. All tests were run using JMP software running on a Macintosh™⃝ IIsi, and significance was accepted at p<0.05.

### II. Results

Topical application of compound 48/80 to the rat mesentery resulted in visible degranulation of more than 90% of the mast cells, which took place within 3 minutes after application. Subsequently, an increase in leukocyte rolling and adhesion was seen. No significant difference in leukocyte accumulation was detected between the 20 and 30 minute timepoints post 48/80 application, the mean of these two timepoints was therefore used in the statistical analysis. The venule-to venule variability in the model was quite high, with baseline interaction ranging from 0 to 4.8 leukocytes present in individual venules, and mast cell-induced interaction ranging from 2.8 to 25.6 leukocytes present in the PBS-treated group (coefficient of variability, CV, 37%). Animal means showed much lower variability (CV, 16%); hence, each individual venule was treated as a separate experimental entity for statistical purposes.

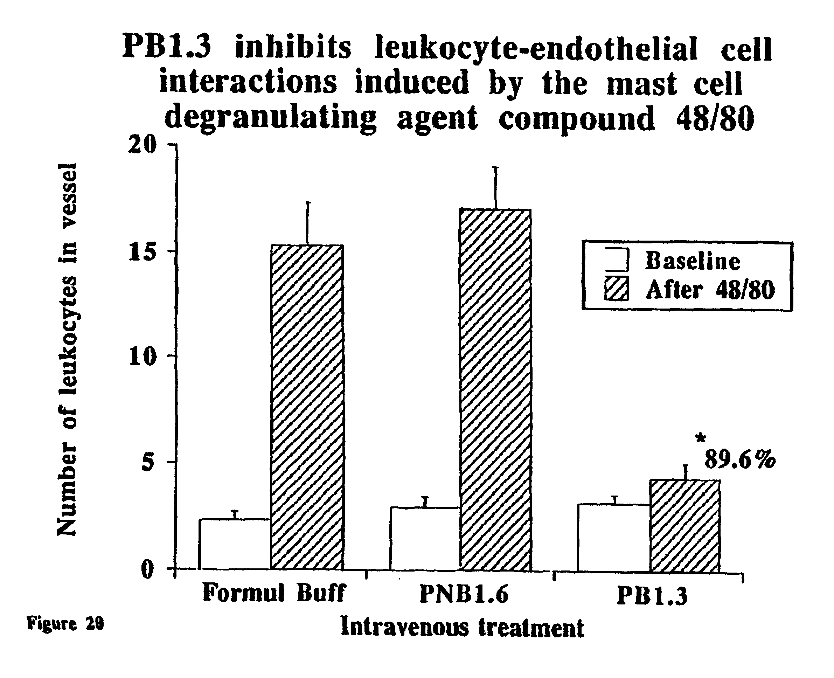

As seen in Fig. 20, intravenous treatment with PB1.3 inhibited the mast cell-induced intravascular leukocyte accumulation by 90% when compared with the formulation buffer control group. The non-reactive antibody PB1.6 did not have any effect in this model.

Thus, administration of the anti-P-selectin antibody, PB1.3, to rats completely prevents the interaction of leukocytes with the vascular endothelium and their subsequent migration into the tissues induced by application of the mast cell degranulating agent 48/80.

## Claims

1. A blocking P-selectin antibody or functional fragment thereof, that competitively inhibits the binding of an antibody secreted by a cell line designated ATCC Accession No. HB11041 to P-selectin as measured by a competitive inhibition assay and wherein said blocking P-selectin antibody, or functional fragment thereof, is capable of binding to P-selectin in the presence of a peptide CQNRYTDLVAIQNKNE and in the absence of Ca²⁺.

2. The blocking P-selectin antibody of claim 1 that inhibits neutrophil binding to P-selectin.

3. The blocking P-selectin antibody according to any one of the preceding claims that is an IgG immunoglobulin.

4. The blocking P-selectin antibody of the preceding claims that is a murine antibody.

5. The blocking P-selectin antibody of any one of claims 1 to 3 that is a human antibody.

6. The blocking P-selectin antibody of any one of claims 1 to 5 that is a humanized or chimeric antibody.

7. The blocking P-selectin antibody according to any one of the preceding claims wherein the functional fragment thereof is Fab, F(ab')₂, CDRs, V_{L}, V_{H} or any combination thereof.

8. Use of a blocking P-selectin antibody according to any one of the preceding claims in the manufacture of a composition for use in a method for treating an inflammatory or thrombotic condition.

9. The use of claim 8, wherein the inflammatory condition is acute lung injury or ischemia reperfusion injury.

10. The use of claim 8 or claim 9, wherein the composition is adapted to be administered intravenously.

11. A pharmaceutical composition comprising a pharmaceutically acceptable carrier and a blocking P-selectin antibody according to any of claims 1 to 7.

12. The use of any one of claims 8 to 10, or the composition of claim 11 wherein the blocking antibody is secreted by the cell line designated ATCC Accession No. HB11041.

13. The use of any one of claims 8 to 10 or the composition of claim 11 wherein the antibody is IgG₁.

14. A blocking P-selectin antibody secreted by a cell line designated ATCC Accession No. HB11041.

15. A composition comprising a cell line designated ATCC Accession No. HB11041.

## Patentansprüche

1. Blockierender P-Selectin-Antikörper oder funktionelles Fragment davon, der die Bindung eines Antikörpers, der von einer Zelllinie mit der ATCC-Hinterlegungsnummer HB 11041 sekretiert wird, an P-Selectin, gemessen mittels eines kompetitiven Hemmungstests, kompetitiv hemmt und worin der blockierende P-Selectin-Antikörper oder das funktionelle Fragment davon in Gegenwart eines Peptids CQNRYTDLVAIQNKNE und in Abwesenheit von Ca²⁺ zur Bindung an P-Selectin fähig ist.

2. Blockierender P-Selectin-Antikörper nach Anspruch 1, der Neutrophil-Bindung an P-Selectin hemmt.

3. Blockierender P-Selectin-Antikörper nach einem der vorangegangenen Ansprüche, der ein IgG-Immunglobulin ist.

4. Blockierender P-Selectin-Antikörper nach einem der vorangegangenen Ansprüche, der ein Mäuse-Antikörper ist.

5. Blockierender P-Selectin-Antikörper nach einem der Ansprüche 1 bis 3, der ein Human-Antikörper ist.

6. Blockierender P-Selectin-Antikörper nach einem der Ansprüche 1 bis 5, der ein humanisierter oder chimärischer Antikörper ist

7. Blockierender P-Selectin-Antikörper nach einem der vorangegangenen Ansprüche, worin sein funktionelles Fragment Fab, F(ab')₂, CDRs, V_{L}, V_{H} oder eine beliebige Kombination davon ist.

8. Verwendung eines blockierenden P-Selectin-Antikörpers nach einem der vorangegangenen Ansprüche zur Herstellung einer Zusammensetzung zur Verwendung in einem Verfahren zur Behandlung einer entzündlichen oder thrombotischen Affektion.

9. Verwendung nach Anspruch 8, worin die entzündliche Affektion akute Lungenschädigung oder Ischämie-Reperfusionsschädigung ist.

10. Verwendung nach Anspruch 8 oder 9, worin die Zusammensetzung an intravenöse Verabreichung angepasst ist.

11. Pharmazeutische Zusammensetzung, die einen pharmazeutisch annehmbaren Träger und einen blockierenden P-Selectin-Antikörper nach einem der Ansprüche 1 bis 7 umfasst.

12. Verwendung nach einem der Ansprüche 8 bis 10 oder Zusammensetzung nach Anspruch 11, worin der blockierende Antikörper von der Zelllinie mit der ATCC-Hinterlegungsnummer HB11041 sekretiert wird.

13. Verwendung nach einem der Ansprüche 8 bis 10 oder Zusammensetzung nach Anspruch 11, worin der Antikörper IgG₁ ist.

14. Von einer Zelllinie mit der ATCC-Hinterlegungsnummer HB11041 sekretierter blockierender P-Selectin-Antikörper.

15. Zusammensetzung, umfassend eine Zelllinie mit der ATCC-Hinterlegungsnummer HB11041.

## Revendications

1. Anticorps bloquant la P-sélectine ou son fragment fonctionnel, qui inhibe compétitivement la liaison d'un anticorps sécrété par une lignée de cellules désignée par le N° d'Accession ATCC HB11041 à la P-sélectine en mesurant par un essai d'inhibition compétitive et où ledit anticorps bloquant la P-sélectine ou son fragment fonctionnel est capable de liaison à la P-sélectine en présence d'un peptide CQNRYTDLVAIQNKNE et en l'absence de Ca²⁺.

2. Anticorps bloquant la P-sélectine de la revendication 1 qui inhibe la liaison des neutrophiles à la P-sélectine.

3. Anticorps bloquant la P-sélectine selon l'une quelconque des revendications précédentes qui est une immunoglobuline IgG.

4. Anticorps bloquant la P-sélectine des revendications précédentes qui est un anticorps de murin.

5. Anticorps bloquant la P-sélectine de l'une quelconque des revendications 1 à 3 qui est un anticorps humain.

6. Anticorps bloquant la P-sélectine de l'une quelconque des revendications 1 à 5 qui est un anticorps humanisé ou chimérique.

7. Anticorps bloquant la P-sélectine selon l'une quelconque des revendications précédentes où le fragment fonctionnel de celui-ci est Fab, F(ab')₂, CDR, V_{L}, V_{H} ou toute combinaison de ceux-ci.

8. Utilisation d'un anticorps bloquant la P-sélectine selon l'une quelconque des revendications précédentes dans la fabrication d'une composition à utiliser dans une méthode pour le traitement d'une condition inflammatoire ou thrombotique.

9. Utilisation de la revendication 8, où la condition inflammatoire est une blessure aiguë des poumons ou une blessure par reperfusion d'ischémie.

10. Utilisation de la revendication 8 ou de la revendication 9, où la composition est adaptée à être administrée par voie intraveineuse.

11. Composition pharmaceutique comprenant un support pharmaceutiquement acceptable et un anticorps bloquant la P-sélectine selon l'une quelconque des revendications 1 à 7.

12. Utilisation de l'une quelconque des revendications 8 à 10 ou de la composition de la revendication 11, où l'anticorps bloquant est sécrété par la lignée de cellules désignée par le N° d'Accession ATCC HB11041.

13. Utilisation de l'une quelconque des revendication 8 à 10 ou de la composition de la revendication 11, où l'anticorps est IgG₁.

14. Anticorps bloquant la P-sélectine sécrété par une lignée de cellules désignée par le N° d'Accession ATCC N° HB11041.

15. Composition comprenant une ligne de cellules désignée par le N° d'Accession ATCC HB11041.


### SEQUENCE LISTING

(1) GENERAL INFORMATION:
   (i) APPLICANT:
      (A) NAME: Cytel Corporation
      (B) STREET: 3525 John Hopkins Court
      (C) CITY: San Diego
      (D) STATE: California
      (E) COUNTRY: USA
      (F) POSTAL CODE (ZIP): 92121
   (ii) TITLE OF INVENTION: Antibodies to P-Selectin and their uses
   (iii) NUMBER OF SEQUENCES: 19
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 93911098.7
   (vi) PRIOR APPLICATION DATA:
      (A) APPLICATION NUMBER: US 07/880,196
      (B) FILING DATE: 05-MAY-1992
(2) INFORMATION FOR SEQ ID NO:1:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Cys Gln Asn Arg Tyr Thr Asp Leu Val Ala Ile Gln Asn Lys Asn Glu
   1               5                   10                  15
(2) INFORMATION FOR SEQ ID NO:2:
   (i) SEQUENCE CHARACTERISTICS:
      (A) LENGTH: 1174 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: join(3..1055, 1059..1142)
   (xi) SEQUENCE DESCRIPTION: SEQ ID NO:2:
   GG ATC CTC TGC GCC TGG GCC CAG CTC TGT CCC ACA CCG CGG TCA CAT      47
      Ile Leu Cys Ala Trp Ala Gln Leu Cys Pro Thr Pro Arg Ser His
       1               5                  10                  15
   GGC ACC ACC TCT CTT GCA GCC TCC ACC AAG GGC CCA TCG GTC TTC CCC     95
   Gly Thr Thr Ser Leu Ala Ala Ser Thr Lys Gly Pro Ser Val Phe Pro
                       20                  25                  30
   CTG GCA CCC TCC TCC AAG AGC ACC TCT GGG GGC ACA GCG GCC CTG GGC    143
   Leu Ala Pro Ser Ser Lys Ser Thr Ser Gly Gly Thr Ala Ala Leu Gly
                   35                  40                  45
   TGC CTG GTC AAG GAC TAC TTC CCC GAA CCG GTG ACG GTG TCG TGG AAC    191
   Cys Leu Val Lys Asp Tyr Phe Pro Glu Pro Val Thr Val Ser Trp Asn
               50                  55                  60
   TCA GGC GCC CTG ACC AGC GGC GTG CAC ACC TTC CCG GCT GTC CTA CAG    239
   Ser Gly Ala Leu Thr Ser Gly Val His Thr Phe Pro Ala Val Leu Gln
           65                  70                  75
   TCC TCA GGA CTC TAC TCC CTC AGC AGC GTG GTG ACC GTG CCC TCC AGC    287
   Ser Ser Gly Leu Tyr Ser Leu Ser Ser Val Val Thr Val Pro Ser Ser
   80                  85                  90                  95
   AGC TTG GGC ACC CAG ACC TAC ATC TGC AAC GTG AAT CAC AAG CCC AGC    335
   Ser Leu Gly Thr Gln Thr Tyr Ile Cys Asn Val Asn His Lys Pro Ser
                   100                 105                 110
   AAC ACC AAG GTG GAC AAG AAA GTT GAG CCC AAA TCT TGT GAC AAA ACT      383
   Asn Thr Lys Val Asp Lys Lys Val Glu Pro Lys Ser Cys Asp Lys Thr
               115                 120                 125
   CAC ACA TGC CCA CCG TGC CCA GCA CCT GAA CTC CTG GGG GGA CCG TCA      431
   His Thr Cys Pro Pro Cys Pro Ala Pro Glu Leu Leu Gly Gly Pro Ser
           130                 135                 140
   GTC TTC CTC TTC CCC CCA AAA CCC AAG GAC ACC CTC ATG ATC TCC CGG      479
   Val Phe Leu Phe Pro Pro Lys Pro Lys Asp Thr Leu Met Ile Ser Arg
       145                 150                 155
   ACC CCT GAG GTC ACA TGC GTG GTG GTG GAC GTG AGC CAC GAA GAC CCT      527
   Thr Pro Glu Val Thr Cys Val Val Val Asp Val Ser His Glu Asp Pro
   160                 165                 170                 175
   GAG GTC AAG TTC AAC TGG TAC GTG GAC GGC GTG GAG GTG CAT AAT GCC      575
   Glu Val Lys Phe Asn Trp Tyr Val Asp Gly Val Glu Val His Asn Ala
                   180                 185                 190
   AAG ACA AAG CCG CGG GAG GAG CAG TAC AAC AGC ACG TAC CGG GTG GTC      623
   Lys Thr Lys Pro Arg Glu Glu Gln Tyr Asn Ser Thr Tyr Arg Val Val
               195                 200                 205
   AGC GTC CTC ACC GTC CTG CAC CAG GAC TGG CTG AAT GGC AAG GAG TAC      671
   Ser Val Leu Thr Val Leu His Gln Asp Trp Leu Asn Gly Lys Glu Tyr
           210                 215                 220
   AAG TGC AAG GTC TCC AAC AAA GCC CTC CCA GCC CCC ATC GAG AAA ACC      719
   Lys Cys Lys Val Ser Asn Lys Ala Leu Pro Ala Pro Ile Glu Lys Thr
       225                 230                 235
   ATC TCC AAA GCC AAA GGG CAG CCC CGA GAA CCA CAG GTG TAC ACC CTG      767
   Ile Ser Lys Ala Lys Gly Gln Pro Arg Glu Pro Gln Val Tyr Thr Leu
   240                 245                 250                 255
   CCC CCA TCC CGG GAT GAG CTG ACC AAG AAC CAG GTC AGC CTG ACC TGC      815
   Pro Pro Ser Arg Asp Glu Leu Thr Lys Asn Gln Val Ser Leu Thr Cys
                   260                 265                 270
   CTG GTC AAA GGC TTC TAT CCC AGC GAC ATC GCC GTG GAG TGG GAG AGC      863
   Leu Val Lys Gly Phe Tyr Pro Ser Asp Ile Ala Val Glu Trp Glu Ser
               275                 280                 285
   AAT GGG CAG CCG GAG AAC AAC TAC AAG ACC ACG CCT CCC GTG CTG GAC      911
   Asn Gly Gln Pro Glu Asn Asn Tyr Lys Thr Thr Pro Pro Val Leu Asp
           290                 295                 300
   TCC GAC GGC TCC TTC TTC CTC TAC AGC AAG CTC ACC GTG GAC AAG AGC      959
   Ser Asp Gly Ser Phe Phe Leu Tyr Ser Lys Leu Thr Val Asp Lys Ser
       305                 310                 315
   AGG TGG CAG CAG GGG AAC GTC TTC TCA TGC TCC GTG ATG CAT GAG GCT     1007
   Arg Trp Gln Gln Gly Asn Val Phe Ser Cys Ser Val Met His Glu Ala
   320                 325                 330                 335
   CTG CAC AAC CAC TAC ACG CAG AAG AGC CTC TCC CTG TCT CCG GGT AAA     1055
   Leu His Asn His Tyr Thr Gln Lys Ser Leu Ser Leu Ser Pro Gly Lys
                   340                 345                 350
   TGA GTG CGA CGG CCG GCA AGC CCC GCT CCC CGG GCT CTC GCG GTC GCA     1103
       Val Arg Arg Pro Ala Ser Pro Ala Pro Arg Ala Leu Ala Val Ala
                   355                 360                 365
   CGA GGA TGC TTG GCA CGT ACC CCC TGT ACA TAC TTC CCG GGCGCCCAGC     1152
   Arg Gly Cys Leu Ala Arg Thr Pro Cys Thr Tyr Phe Pro
               370                 375
   ATGGAAATAA AGCACCGGAT CC                                           1174
(2) INFORMATION FOR SEQ ID NO:3:
   (i) SEQUENCE CHARACTERISTICS:
      (A) LENGTH: 379 amino acids
      (B) TYPE: amino acid
      (D) TOPOLOGY: linear
   (ii) MOLECULE TYPE; protein
   (xi) SEQUENCE DESCRIPTION: SEQ ID NO:3:
   Ile Leu Cys Ala Trp Ala Gln Leu Cys Pro Thr Pro Arg Ser His Gly
    1               5                  10                  15
   Thr Thr Ser Leu Ala Ala Ser Thr Lys Gly Pro Ser Val Phe Pro Leu
               20                  25                  30
   Ala Pro Ser Ser Lys Ser Thr Ser Gly Gly Thr Ala Ala Leu Gly Cys
   35 40 45
   Leu Val Lys Asp Tyr Phe Pro Glu Pro Val Thr Val Ser Trp Asn Ser
   50 55 60
   Gly Ala Leu Thr Ser Gly Val His Thr Phe Pro Ala Val Leu Gln Ser
   65 70 75 80
   Ser Gly Leu Tyr Ser Leu Ser Ser Val Val Thr Val Pro Ser Ser Ser
   85 90 95
   Leu Gly Thr Gln Thr Tyr Ile Cys Asn Val Asn His Lys Pro Ser Asn
   100 105 110
   Thr Lys Val Asp Lys Lys Val Glu Pro Lys Ser Cys Asp Lys Thr His
   115 120 125
   Thr Cys Pro Pro Cys Pro Ala Pro Glu Leu Leu Gly Gly Pro Ser Val
   130 135 140
   Phe Leu Phe Pro Pro Lys Pro Lys Asp Thr Leu Met Ile Ser Arg Thr
   145 150 155 160
   Pro Glu Val Thr Cys Val Val Val Asp Val Ser His Glu Asp Pro Glu
   165 170 175
   Val Lys Phe Asn Trp Tyr Val Asp Gly Val Glu Val His Asn Ala Lys
   180 185 190
   Thr Lys Pro Arg Glu Glu Gln Tyr Asn Ser Thr Tyr Arg Val Val Ser
   195 200 205
   Val Leu Thr Val Leu His Gln Asp Trp Leu Asn Gly Lys Glu Tyr Lys
   210 215 220
   Cys Lys Val Ser Asn Lys Ala Leu Pro Ala Pro Ile Glu Lys Thr Ile
   225 230 235 240
   Ser Lys Ala Lys Gly Gln Pro Arg Glu Pro Gln Val Tyr Thr Leu Pro
   245 250 255
   Pro Ser Arg Asp Glu Leu Thr Lys Asn Gln Val Ser Leu Thr Cys Leu
   260 265 270
   Val Lys Gly Phe Tyr Pro Ser Asp Ile Ala Val Glu Trp Glu Ser Asn
   275 280 285
   Gly Gln Pro Glu Asn Asn Tyr Lys Thr Thr Pro Pro Val Leu Asp Ser
   290 295 300
   Asp Gly Ser Phe Phe Leu Tyr Ser Lys Leu Thr Val Asp Lys Ser Arg
   305 310 315 320
   Trp Gln Gln Gly Asn Val Phe Ser Cys Ser Val Met His Glu Ala Leu
   325 330 335
   His Asn His Tyr Thr Gln Lys Ser Leu Ser Leu Ser Pro Gly Lys Val
   340 345 350
   Arg Arg Pro Ala Ser Pro Ala Pro Arg Ala Leu Ala Val Ala Arg Gly
   355 360 365
   Cys Leu Ala Arg Thr Pro Cys Thr Tyr Phe Pro
   370 375
(2) INFORMATION FOR SEQ ID NO:4:
   (i) SEQUENCE CHARACTERISTICS:
      (A) LENGTH: 318 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: 1..318
   (xi) SEQUENCE DESCRIPTION: SEQ ID NO:4:
   ACT GTG GCT GCA CCA TCT GTC TTC ATC TTC CCG CCA TCT GAT GAG CAG 48
   Thr Val Ala Ala Pro Ser Val Phe Ile Phe Pro Pro Ser Asp Glu Gln
   1 5 10 15
   TTG AAA TCT GGA ACT GCC TCT GTT GTG TGC CTG CTG AAT AAC TTC TAT 96
   Leu Lys Ser Gly Thr Ala Ser Val Val Cys Leu Leu Asn Asn Phe Tyr
   20 25 30
   CCC AGA GAG GCC AAA GTA CAG TGG AAG GTG GAT AAC GCC CTC CAA TCG      144
   Pro Arg Glu Ala Lys Val Gln Trp Lys Val Asp Asn Ala Leu Gln Ser
            35                  40                  45
   GGT AAC TCC CAG GAG AGT GTC ACA GAG CAG GAC AGC AAG GAC AGC ACC      192
   Gly Asn Ser Gln Glu Ser Val Thr Glu Gln Asp Ser Lys Asp Ser Thr
        50                  55                  60
   TAC AGC CTC AGC AGC ACC CTG ACG CTG AGC AAA GCA GAC TAC GAG AAA      240
   Tyr Ser Leu Ser Ser Thr Leu Thr Leu Ser Lys Ala Asp Tyr Glu Lys
    65                  70                  75                  80
   CAC AAA GTC TAC GCC TGC GAA GTC ACC CAT CAG GGC CTG AGC TCG CCC      288
   His Lys Val Tyr Ala Cys Glu Val Thr His Gln Gly Leu Ser Ser Pro
                    85                  90                  95
   GTC ACA AAG AGC TTC AAC AGG GGA GAG TGT                              318
   Val Thr Lys Ser Phe Asn Arg Gly Glu Cys
               100                 105
(2) INFORMATION FOR SEQ ID NO:5:
   (i) SEQUENCE CHARACTERISTICS:
      (A) LENGTH: 106 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Thr Val Ala Ala Pro Ser Val Phe Ile Phe Pro Pro Ser Asp Glu Gln
     1               5                  10                  15
   Leu Lys Ser Gly Thr Ala Ser Val Val Cys Leu Leu Asn Asn Phe Tyr
                20                  25                  30
   Pro Arg Glu Ala Lys Val Gln Trp Lys Val Asp Asn Ala Leu Gln Ser
            35                  40                  45
   Gly Asn Ser Gln Glu Ser Val Thr Glu Gln Asp Ser Lys Asp Ser Thr
        50                  55                  60
   Tyr Ser Leu Ser Ser Thr Leu Thr Leu Ser Lys Ala Asp Tyr Glu Lys
    65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
(2) INFORMATION FOR SEQ ID NO:6:
   (i) SEQUENCE CHARACTERISTICS:
      (A) LENGTH: 342 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..336
   (xi) SEQUENCE DESCRIPTION: SEQ ID NO:6:
   ATG GGA TGG AGC GGG GTC TTT CTC TTT CTC CTG TCA GGA ACT GCA GGT      48
   Met Gly Trp Ser Gly Val Phe Leu Phe Leu Leu Ser Gly Thr Ala Gly
     1               5                  10                  15
   GTC CAC TCT GAG GCC CAG CTG CAG CAG TCT GGA CCT GAG CTG GTA GAG      96
   Val His Ser Glu Ala Gln Leu Gln Gln Ser Gly Pro Glu Leu Val Glu
                20                  25                  30
   CCT GGG GCT TCA GTG AAG GTG TCC TGC AAG GCT TCT GGA TAC ACA TTC     144
   Pro Gly Ala Ser Val Lys Val Ser Cys Lys Ala Ser Gly Tyr Thr Phe
            35                  40                  45
   ACC AAC TAT GTT ATG CAC TGG GTG AAG CAG AAG CCT GGG CAG GGC CTT     192
   Thr Asn Tyr Val Met His Trp Val Lys Gln Lys Pro Gly Gln Gly Leu
        50                  55                  60
   GAG TGG ATT GGA TTT ATT AAC CCA TCC AAT GAT GGT CCT AAG TAC AAT     240
   Glu Trp Ile Gly Phe Ile Asn Pro Ser Asn Asp Gly Pro Lys Tyr Asn
    65                  70                  75                  80
   GAG AGG TTC AAA AAC AAG GCC ACA CTG ACT TCA GAC AAA TCC TCC AGC      288
   Glu Arg Phe Lys Asn Lys Ala Thr Leu Thr Ser Asp Lys Ser Ser Ser
                   85                  90                  95
   ACA GCC TAC ATG GAG CTC AGC AGC CTG ACC TCT GAG GTC TCT GCG GTC      336
   Thr Ala Tyr Met Glu Leu Ser Ser Leu Thr Ser Glu Val Ser Ala Val
               100                 105                 110
   TATTTC                                                               342
(2) INFORMATION FOR SEQ ID NO:7:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO:7:
   Met Gly Trp Ser Gly Val Phe Leu Phe Leu Leu Ser Gly Thr Ala Gly
    1               5                  10                  15
   Val His Ser Glu Ala Gln Leu Gln Gln Ser Gly Pro Glu Leu Val Glu
               20                  25                  30
   Pro Gly Ala Ser Val Lys Val Ser Cys Lys Ala Ser Gly Tyr Thr Phe
           35                  40                  45
   Thr Asn Tyr Val Met His Trp Val Lys Gln Lys Pro Gly Gln Gly Leu
       50                  55                  60
   Glu Trp Ile Gly Phe Ile Asn Pro Ser Asn Asp Gly Pro Lys Tyr Asn
   65                  70                  75                  80
   Glu Arg Phe Lys Asn Lys Ala Thr Leu Thr Ser Asp Lys Ser Ser Ser
                   85                  90                  95
   Thr Ala Tyr Met Glu Leu Ser Ser Leu Thr Ser Glu Val Ser Ala Val
               100                 105                 110
(2) INFORMATION FOR SEQ ID NO:8:
   (i) SEQUENCE CHARACTERISTICS:
      (A) LENGTH: 336 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..336
   (xi) SEQUENCE DESCRIPTION: SEQ ID NO:8:
   ATG GGC ATC AAG ATG GAG TCA CAG ATT CAG GTC TTT GTA TAC ATG TTG       48
   Met Gly Ile Lys Met Glu Ser Gln Ile Gln Val Phe Val Tyr Met Leu
    1               5                  10                  15
   CTG TGG TTG TCT GGT GTT GAT GGA GAC ATT GTG ATG ACC CAG TCT CAA       96
   Leu Trp Leu Ser Gly Val Asp Gly Asp Ile Val Met Thr Gln Ser Gln
                20                  25                  30
   AAA TTC ATG TCC ACA TCA GTA GGA GAC AGG GTC AGC GTC ACC TGC AAG      144
   Lys Phe Met Ser Thr Ser Val Gly Asp Arg Val Ser Val Thr Cys Lys
            35                  40                  45
   GCC AGT CAG AAT GTG GCT ACT AAT GTA GTC TGG TAT CAA CAG AGA CCA      192
   Ala Ser Gln Asn Val Ala Thr Asn Val Val Trp Tyr Gln Gln Arg Pro
        50                  55                  60
   GGA CAA TCT CCT AAA GCG CTT ATT TAT ACG GCA TCC TAC CGG TTC AGT      240
   Gly Gln Ser Pro Lys Ala Leu Ile Tyr Thr Ala Ser Tyr Arg Phe Ser
    65                  70                  75                  80
   GGA GTC CCT GAA CGC TTC TCA GGC AGT GGA TCT GGG ACA GAT TTC ACT      288
   Gly Val Pro Glu Arg Phe Ser Gly Ser Gly Ser Gly Thr Asp Phe Thr
                    85                  90                  95
   CTC ACC ATC ACC AAT GTG CAG TCT GAA GAC TTG GCA GAC TAT TTC TGT      336
   Leu Thr Ile Thr Asn Val Gln Ser Glu Asp Leu Ala Asp Tyr Phe Cys
               100                 105                 110
(2) INFORMATION FOR SEQ ID NO:9:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO:9:
   Met Gly Ile Lys Met Glu Ser Gln Ile Gln Val Phe Val Tyr Met Leu
    1               5                  10                  15
   Leu Trp Leu Ser Gly Val Asp Gly Asp Ile Val Met Thr Gln Ser Gln
               20                  25                  30
   Lys Phe Met Ser Thr Ser Val Gly Asp Arg Val Ser Val Thr Cys Lys
           35                  40                  45
   Ala Ser Gln Asn Val Ala Thr Asn Val Val Trp Tyr Gln Gln Arg Pro
       50                  55                  60
   Gly Gln Ser Pro Lys Ala Leu Ile Tyr Thr Ala Ser Tyr Arg Phe Ser
   65                  70                  75                  80
   Gly Val Pro Glu Arg Phe Ser Gly Ser Gly Ser Gly Thr Asp Phe Thr
                   85                  90                  95
   Leu Thr Ile Thr Asn Val Gln Ser Glu Asp Leu Ala Asp Tyr Phe Cys
               100                 105                 110
(2) INFORMATION FOR SEQ ID NO:10:
   (i) SEQUENCE CHARACTERISTICS:
      (A) LENGTH: 417 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..417
   (xi) SEQUENCE DESCRIPTION: SEQ ID NO:10:
   ATG GAG TTT GGG CTG AGC TGG CTT TTT CTT GTG GCT ATT TTA AAA GGT      48
   Met Glu Phe Gly Leu Ser Trp Leu Phe Leu Val Ala Ile Leu Lys Gly
    1               5                  10                  15
   GTC CAG TGT GAG GCC CAG CTG GTG CAG TCT GGA GCT GAG GTG AAA AAG      96
   Val Gln Cys Glu Ala Gln Leu Val Gln Ser Gly Ala Glu Val Lys Lys
               20                  25                  30
   CCT GGG GCT TCA GTG AAG GTG TCC TGC AAG GCT TCT GGA TAC ACA TTC     144
   Pro Gly Ala Ser Val Lys Val Ser Cys Lys Ala Ser Gly Tyr Thr Phe
           35                  40                  45
   ACC AAC TAT GTT ATG CAC TGG GTG CGC CAA GCT CCC GGG CAG AGG CTT     192
   Thr Asn Tyr Val Met His Trp Val Arg Gln Ala Pro Gly Gln Arg Leu
       50                  55                  60
   GAG TGG ATG GGA TTT ATT AAC CCA TCC AAT GAT GGT CCT AAG TAC AAT     240
   Glu Trp Met Gly Phe Ile Asn Pro Ser Asn Asp Gly Pro Lys Tyr Asn
   65                  70                  75                  80
   GAG AGG TTC AAA AAC AGG GTC ACA ATC ACT TCA GAC ACA TCC GCC AGC     288
   Glu Arg Phe Lys Asn Arg Val Thr Ile Thr Ser Asp Thr Ser Ala Ser
                   85                  90                  95
   ACC GCC TAC ATG GAA CTG TCC AGC CTG CGC TCC GAG GAC ACT GCA GTC     336
   Thr Ala Tyr Met Glu Leu Ser Ser Leu Arg Ser Glu Asp Thr Ala Val
               100                 105                 110
   TAT TAC TGT GCC AGA GCA CGC CCG GGG TTC GAC TGG TAC TTC GAT GTC     384
   Tyr Tyr Cys Ala Arg Ala Arg Pro Gly Phe Asp Trp Tyr Phe Asp Val
           115                 120                 125
   TGG GGA CAG GGT ACC CTT GTC ACC GTC AGT TCA                         417
   Trp Gly Gln Gly Thr Leu Val Thr Val Ser Ser
       130                 135
(2) INFORMATION FOR SEQ ID NO:11:
   (i) SEQUENCE CHARACTERISTICS:
      (A) LENGTH: 139 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Glu Phe Gly Leu Ser Trp Leu Phe Leu Val Ala Ile Leu Lys Gly
   1 5 10 15
   Val Gln Cys Glu Ala Gln Leu Val Gln Ser Gly Ala Glu Val Lys Lys
   20 25 30
   Pro Gly Ala Ser Val Lys Val Ser Cys Lys Ala Ser Gly Tyr Thr Phe
   35 40 45
   Thr Asn Tyr Val Met His Trp Val Arg Gln Ala Pro Gly Gln Arg Leu
   50 55 60
   Glu Trp Met Gly Phe Ile Asn Pro Ser Asn Asp Gly Pro Lys Tyr Asn
   65 70 75 80
   Glu Arg Phe Lys Asn Arg Val Thr Ile Thr Ser Asp Thr Ser Ala Ser
   85 90 95
   Thr Ala Tyr Met Glu Leu Ser Ser Leu Arg Ser Glu Asp Thr Ala Val
   100 105 110
   Tyr Tyr Cys Ala Arg Ala Arg Pro Gly Phe Asp Trp Tyr Phe Asp Val
   115 120 125
   Trp Gly Gln Gly Thr Leu Val Thr Val Ser Ser
   130 135
(2) INFORMATION FOR SEQ ID NO:12:
   (i) SEQUENCE CHARACTERISTICS:
      (A) LENGTH: 336 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..336
   (xi) SEQUENCE DESCRIPTION: SEQ ID NO:12:
   ATG GAG TTT GGG CTG AGC TGG CTT TTT CTT GTG GCT ATT TTA AAA GGT     48
   Met Glu Phe Gly Leu Ser Trp Leu Phe Leu Val Ala Ile Leu Lys Gly
    1               5                  10                  15
   GTC CAG TGT CAG GTC CAG CTG GTG CAG TCT GGA GCT GAG GTG AAA AAG     96
   Val Gln Cys Gln Val Gln Leu Val Gln Ser Gly Ala Glu Val Lys Lys
               20                  25                  30
   CCT GGG GCT TCA GTG AAG GTG TCC TGC AAG GCT TCT GGA TAC ACA TTC    144
   Pro Gly Ala Ser Val Lys Val Ser Cys Lys Ala Ser Gly Tyr Thr Phe
           35                  40                  45
   ACC AAC TAT GTT ATG CAC TGG GTG CGC CAA GCT CCC GGG CAG AGG CTT    192
   Thr Asn Tyr Val Met His Trp Val Arg Gln Ala Pro Gly Gln Arg Leu
       50                  55                  60
   GAG TGG ATG GGA TTT ATT AAC CCA TCC AAT GAT GGT CCT AAG TAC AAT    240
   Glu Trp Met Gly Phe Ile Asn Pro Ser Asn Asp Gly Pro Lys Tyr Asn
   65                  70                  75                  80
   GAG AGG TTC AAA AAC AGG GTC ACA ATC ACT TCA GAC ACA TCC GCC AGC    288
   Glu Arg Phe Lys Asn Arg Val Thr Ile Thr Ser Asp Thr Ser Ala Ser
                   85                  90                  95
   ACC GCC TAC ATG GAA CTG TCC AGC CTG CGC TCC GAG GAC ACT GCA GTC    336
   Thr Ala Tyr Met Glu Leu Ser Ser Leu Arg Ser Glu Asp Thr Ala Val
               100                 105                 110
(2) INFORMATION FOR SEQ ID NO:13:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Glu Phe Gly Leu Ser Trp Leu Phe Leu Val Ala Ile Leu Lys Gly
     1               5                  10                  15
   Val Gln Cys Gln Val Gln Leu Val Gln Ser Gly Ala Glu Val Lys Lys
                20                  25                  30
   Pro Gly Ala Ser Val Lys Val Ser Cys Lys Ala Ser Gly Tyr Thr Phe
            35                  40                  45
   Thr Asn Tyr Val Met His Trp Val Arg Gln Ala Pro Gly Gln Arg Leu
        50                  55                  60
   Glu Trp Met Gly Phe Ile Asn Pro Ser Asn Asp Gly Pro Lys Tyr Asn
    65                  70                  75                  80
   Glu Arg Phe Lys Asn Arg Val Thr Ile Thr Ser Asp Thr Ser Ala Ser
                    85                  90                  95
   Thr Ala Tyr Met Glu Leu Ser Ser Leu Arg Ser Glu Asp Thr Ala Val
               100                 105                 110
(2) INFORMATION FOR SEQ ID NO:14:
   (i) SEQUENCE CHARACTERISTICS:
      (A) LENGTH: 417 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..417
   (xi) SEQUENCE DESCRIPTION: SEQ ID NO:14:
   ATG GAG TTT GGG CTG AGC TGG CTT TTT CTT GTG GCT ATT TTA AAA GGT        48
   Met Glu Phe Gly Leu Ser Trp Leu Phe Leu Val Ala Ile Leu Lys Gly
     1               5                  10                  15
   GTC CAG TGT GAG GCC CAG CTG GTG CAG TCT GGA GCT GAG GTG AAA AAG        96
   Val Gln Cys Glu Ala Gln Leu Val Gln Ser Gly Ala Glu Val Lys Lys
               20                  25                  30
   CCT GGG GCT TCA GTG AAG GTG TCC TGC AAG GCT TCT GGA TAC ACA TTC       144
   Pro Gly Ala Ser Val Lys Val Ser Cys Lys Ala Ser Gly Tyr Thr Phe
           35                  40                  45
   ACC AAC TAT GTT ATG CAC TGG GTG CGC CAA GCT CCC GGG CAG AGG CTT       192
   Thr Asn Tyr Val Met His Trp Val Arg Gln Ala Pro Gly Gln Arg Leu
       50                  55                  60
   GAG TGG ATG GGA TTT ATT AAC CCA TCC AAT GAT GGT CCT AAG TAC AAT       240
   Glu Trp Met Gly Phe Ile Asn Pro Ser Asn Asp Gly Pro Lys Tyr Asn
   65                  70                  75                  80
   GAG AGG TTC AAA AAC AGG GTC ACA ATC ACT TCA GAC AAA TCC GCC AGC       288
   Glu Arg Phe Lys Asn Arg Val Thr Ile Thr Ser Asp Lys Ser Ala Ser
                   85                  90                  95
   ACC GCC TAC ATG GAA CTG TCC AGC CTG CGC TCC GAG GAC ACT GCA GTC       336
   Thr Ala Tyr Met Glu Leu Ser Ser Leu Arg Ser Glu Asp Thr Ala Val
               100                 105                 110
   TAT TAC TGT GCC AGA GCA CGC CCG GGG TTC GAC TGG TAC TTC GAT GTC       384
   Tyr Tyr Cys Ala Arg Ala Arg Pro Gly Phe Asp Trp Tyr Phe Asp Val
           115                 120                 125
   TGG GGA CAG GGT ACC CTT GTC ACC GTC AGT TCA                           417
   Trp Gly Gln Gly Thr Leu Val Thr Val Ser Ser
       130                 135
(2) INFORMATION FOR SEQ ID NO:15:
   (i) SEQUENCE CHARACTERISTICS:
      (A) LENGTH: 139 amino acids
      (B) TYPE: amino acid
      (D) TOPOLOGY: linear
   (ii) MOLECULE TYPE: protein
   (xi) SEQUENCE DESCRIPTION: SEQ ID NO:15:
   Met Glu Phe Gly Leu Ser Trp Leu Phe Leu Val Ala Ile Leu Lys Gly
   1               5                   10                  15
   Val Gln Cys Glu Ala Gln Leu Val Gln Ser Gly Ala Glu Val Lys Lys
   20 25 30
   Pro Gly Ala Ser Val Lys Val Ser Cys Lys Ala Ser Gly Tyr Thr Phe
   35 40 45
   Thr Asn Tyr Val Met His Trp Val Arg Gln Ala Pro Gly Gln Arg Leu
   50 55 60
   Glu Trp Met Gly Phe Ile Asn Pro Ser Asn Asp Gly Pro Lys Tyr Asn
   65 70 75 80
   Glu Arg Phe Lys Asn Arg Val Thr Ile Thr Ser Asp Lys Ser Ala Ser
   85 90 95
   Thr Ala Tyr Met Glu Leu Ser Ser Leu Arg Ser Glu Asp Thr Ala Val
   100 105 110
   Tyr Tyr Cys Ala Arg Ala Arg Pro Gly Phe Asp Trp Tyr Phe Asp Val
   115 120 125
   Trp Gly Gln Gly Thr Leu Val Thr Val Ser Ser
   130 135
(2) INFORMATION FOR SEQ ID NO:16:
   (i) SEQUENCE CHARACTERISTICS:
      (A) LENGTH: 378 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..378
   (xi) SEQUENCE DESCRIPTION: SEQ ID NO:16:
   ATG GGA TGG AGC TGT ATC ATC CTC TTC TTG GTA GCA ACA GCT ACA GGT 48
   Met Gly Trp Ser Cys Ile Ile Leu Phe Leu Val Ala Thr Ala Thr Gly
   1 5 10 15
   GTC CAC TCC GAC ATC CAG ATG ACC CAG AGC CCA TCC ACC CTG TCT GCA          96
   Val His Ser Asp Ile Gln Met Thr Gln Ser Pro Ser Thr Leu Ser Ala
               20                  25                  30
   AGC GTA GGA GAC AGA GTC ACC GTC ACC TGC AAG GCC AGT CAG AAT GTG         144
   Ser Val Gly Asp Arg Val Thr Val Thr Cys Lys Ala Ser Gln Asn Val
           35                  40                  45
   GCT ACT AAT GTA GTC TGG TAT CAA CAG AAA CCA GGA GAG GCT CCT AAA         192
   Ala Thr Asn Val Val Trp Tyr Gln Gln Lys Pro Gly Glu Ala Pro Lys
       50                  55                  60
   GCG CTT ATT TAT ACG GCA TCC TAC CGG TTC AGT GGA GTC CCT GAA CGC         240
   Ala Leu Ile Tyr Thr Ala Ser Tyr Arg Phe Ser Gly Val Pro Glu Arg
   65                  70                  75                  80
   TTC TCA GGC AGT GGA TCT GGG ACA GAT TTC ACT CTC ACC ATC AGC AGT         288
   Phe Ser Gly Ser Gly Ser Gly Thr Asp Phe Thr Leu Thr Ile Ser Ser
                   85                  90                  95
   CTG CAG TCT GAT GAC TTT GCA ACT TAT TAC TGT CAA CAG TAT AAC AAT         336
   Leu Gln Ser Asp Asp Phe Ala Thr Tyr Tyr Cys Gln Gln Tyr Asn Asn
               100                 105                 110
   TAC CCA TAC ACG TTC GGC CAA GGG ACC AAG CTC GAA ATC AAA                 378
   Tyr Pro Tyr Thr Phe Gly Gln Gly Thr Lys Leu Glu Ile Lys
           115                 120                 125
(2) INFORMATION FOR SEQ ID NO:17:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Gly Trp Ser Cys Ile Ile Leu Phe Leu Val Ala Thr Ala Thr Gly
    1               5                  10                  15
   Val His Ser Asp Ile Gln Met Thr Gln Ser Pro Ser Thr Leu Ser Ala
               20                  25                  30
   Ser Val Gly Asp Arg Val Thr Val Thr Cys Lys Ala Ser Gln Asn Val
   35 40 45
   Ala Thr Asn Val Val Trp Tyr Gln Gln Lys Pro Gly Glu Ala Pro Lys
   50 55 60
   Ala Leu Ile Tyr Thr Ala Ser Tyr Arg Phe Ser Gly Val Pro Glu Arg
   65 70 75 80
   Phe Ser Gly Ser Gly Ser Gly Thr Asp Phe Thr Leu Thr Ile Ser Ser
   85 90 95
   Leu Gln Ser Asp Asp Phe Ala Thr Tyr Tyr Cys Gln Gln Tyr Asn Asn
   100 105 110
   Tyr Pro Tyr Thr Phe Gly Gln Gly Thr Lys Leu Glu Ile Lys
   115 120 125
(2) INFORMATION FOR SEQ ID NO:18:
   (i) SEQUENCE CHARACTERISTICS:
      (A) LENGTH: 378 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..378
   (xi) SEQUENCE DESCRIPTION: SEQ ID NO:18:
   ATG GGA TGG AGC TGT ATC ATC CTC TTC TTG GTA GCA ACA GCT ACA GGT 48
   Met Gly Trp Ser Cys Ile Ile Leu Phe Leu Val Ala Thr Ala Thr Gly
   1 5 10 15
   GTC CAC TCC GAC ATC CAG ATG ACC CAG AGC CCA TCC ACC CTG TCT GCA 96
   Val His Ser Asp Ile Gln Met Thr Gln Ser Pro Ser Thr Leu Ser Ala
   20 25 30
   AGC GTA GGA GAC AGA GTC ACC GTC ACC TGC AAG GCC AGT CAG AAT GTG 144
   Ser Val Gly Asp Arg Val Thr Val Thr Cys Lys Ala Ser Gln Asn Val
   35 40 45
   GCT ACT AAT GTA GTC TGG TAT CAA CAG AAA CCA GGA GAG GCT CCT AAA        192
   Ala Thr Asn Val Val Trp Tyr Gln Gln Lys Pro Gly Glu Ala Pro Lys
        50                  55                  60
   GCG CTT ATT TAT ACG GCA TCC TAC CGG TTC AGT GGA GTC CCT TCA CGC        240
   Ala Leu Ile Tyr Thr Ala Ser Tyr Arg Phe Ser Gly Val Pro Ser Arg
    65                  70                  75                  80
   TTC TCA GGC AGT GGA TCT GGG ACA GAT TTC ACT CTC ACC ATC AGC AGT        288
   Phe Ser Gly Ser Gly Ser Gly Thr Asp Phe Thr Leu Thr Ile Ser Ser
                    85                  90                  95
   CTG CAG TCT GAT GAC TTT GCA ACT TAT TAC TGT CAA CAG TAT AAC AAT        336
   Leu Gln Ser Asp Asp Phe Ala Thr Tyr Tyr Cys Gln Gln Tyr Asn Asn
               100                 105                 110
   TAC CCA TAC ACG TTC GGC CAA GGG ACC AAG CTC GAA ATC AAA                378
   Tyr Pro Tyr Thr Phe Gly Gln Gly Thr Lys Leu Glu Ile Lys
           115                 120                 125
(2) INFORMATION FOR SEQ ID NO:19:
   (i) SEQUENCE CHARACTERISTICS:
      (A) LENGTH: 126 amino acids
      (B) TYPE: amino acid
      (D) TOPOLOGY: linear
   (ii) MOLECULE TYPE: protein
   (xi) SEQUENCE DESCRIPTION: SEQ ID NO:19:
   Met Gly Trp Ser Cys Ile Ile Leu Phe Leu Val Ala Thr Ala Thr Gly
     1               5                  10                  15
   Val His Ser Asp Ile Gln Met Thr Gln Ser Pro Ser Thr Leu Ser Ala
                20                  25                  30
   Ser Val Gly Asp Arg Val Thr Val Thr Cys Lys Ala Ser Gln Asn Val
            35                  40                  45
   Ala Thr Asn Val Val Trp Tyr Gln Gln Lys Pro Gly Glu Ala Pro Lys
        50                  55                  60
   Ala Leu Ile Tyr Thr Ala Ser Tyr Arg Phe Ser Gly Val Pro Ser Arg
    65                  70                  75                  80
   Phe Ser Gly Ser Gly Ser Gly Thr Asp Phe Thr Leu Thr Ile Ser Ser
   85 90 95
   Leu Gln Ser Asp Asp Phe Ala Thr Tyr Tyr Cys Gln Gln Tyr Asn Asn
   100 105 110
   Tyr Pro Tyr Thr Phe Gly Gln Gly Thr Lys Leu Glu Ile Lys
   115 120 125